(19) Europäisches Patentamt
European Patent Office
Office européen des brevets

(11) Veröffentlichungsnummer : **0 398 096 B1**

(12) **EUROPÄISCHE PATENTSCHRIFT**

(45) Veröffentlichungstag der Patentschrift : **13.10.93 Patentblatt 93/41**

(51) Int. Cl.[5] : **C07D 249/14,** A01N 43/653

(21) Anmeldenummer : **90108482.2**

(22) Anmeldetag : **05.05.90**

(54) **Substituierte Triazolone.**

Die Akte enthält technische Angaben, die nach dem Eingang der Anmeldung eingereicht wurden und die nicht in dieser Patentschrift enthalten sind.

(30) Priorität : **18.05.89 DE 3916208**

(43) Veröffentlichungstag der Anmeldung : **22.11.90 Patentblatt 90/47**

(45) Bekanntmachung des Hinweises auf die Patenterteilung : **13.10.93 Patentblatt 93/41**

(84) Benannte Vertragsstaaten : **BE CH DE FR GB IT LI NL**

(56) Entgegenhaltungen :
**EP-A- 0 283 876**
**GB-A- 919 458**

(56) Entgegenhaltungen :
**CHEMICAL ABSTRACTS, Band 77, Nr. 22, 27. November 1972, Seite 507, Zusammenfassung Nr. 147067a, Columbus, Ohio, US; & 9TH COLLECTIVE FORMULA INDEX, (1972-1976), Seite 1831f, linke Spalte, unter C4H12N4S, 3H-1,2,4-Triazole-3-thione, 4-ethyl-S-(ethylamino-)-2,4-dihydro; & JP-A-71 37 646 (METAL TREATING TECHNOLOGY)**

(73) Patentinhaber : **BAYER AG**
**D-51368 Leverkusen (DE)**

(72) Erfinder : **Findeisen, Kurt, Dr.**
**Dünfelder Strasse 28**
**D-5090 Leverkusen 1 (DE)**
Erfinder : **Lindig, Markus, Dr.**
**Dahlienweg 16**
**D-4010 Hilden (DE)**
Erfinder : **Santel, Hans-Joachim, Dr.**
**Grünstrasse 9a**
**D-5090 Leverkusen 1 (DE)**
Erfinder : **Schmidt, Robert R., Dr.**
**Im Waldwinkel 110**
**D-5060 Bergisch Gladbach 2 (DE)**
Erfinder : **Lürssen, Klaus, Dr.**
**August-Kierspel-Strasse 145**
**D-5060 Bergisch Gladbach 2 (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue substituierte Triazolone, mehrere Verfahren zu ihrer Herstellung und ihre Verwendung als Herbizide.

Es ist bekannt, daß bestimmte substituierte Triazolone wie beispielsweise die Verbindung 1-(Cyclohexylaminocarbonyl)-3-dimethylamino-4-methyl-1,2,4-triazolin-5-on herbizide Eigenschaften besitzen (vgl. z.B. EP 283 876).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Es wurden neue substituierte Triazolone der allgemeinen Formel (I),

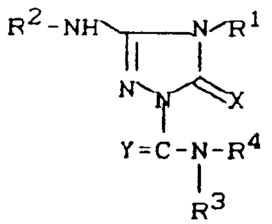

(I)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten infrage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlen- stoffatomen, Halogenalkyl mit 1 bis 8 Kohlenstoff- atomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl mit jeweils 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil, Aryl mit 6 bis 10 Kohlenstoffatomen steht, wobei als Substituenten im Arylteil jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^3$ und $R^4$ unabhängig voneinander jeweils für Wasserstoff, für jeweils geradkettiges oder verzweigtes Alkyl mit 1 bis 18 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, für Alkylaminoalkyl, Dialkylaminoalkyl oder Alkoximinoalkyl mit jeweils 1 bis 8 Kohlenstoffatomen in den einzelnen Alkylteilen oder für jeweils

gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenene Halogenatomen oder jeweils zweifach verknüpftes Alkandiyl oder Alkendiyl mit jeweils bis zu 4 Kohlenstoffatomen; $R^3$ und $R^4$ außerdem unabhangig voneinander für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil stehen, wobei als Substituenten infrage kommen:

Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^3$ und $R^4$ außerdem unabhängig voneinander für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aralkyloxy, Aryloxy, Aroyl oder Aryl mit jeweils 6 bis 10 Kohlenstoffatomen im Arylteil und gegebenenfalls 1 bis 6 Kohlenstoffatomen im Alkylteil stehen, wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen oder Phenoxy und wobei als Alkylsubstituenten gegebenenfalls infrage kommen: Halogen oder Cyano, oder

$R^3$ und $R^4$ gemeinsam mit dem Stickstoffatom, an welches sie gebunden sind, für einen gegebenenfalls einfach oder mehrfach, gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel, enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen,

X für Sauerstoff oder Schwefel steht und

Y für Sauerstoff oder Schwefel steht, gefunden.

Weiterhin wurde gefunden, daß man die neuen substituierten Triazolone der allgemeinen Formel (I),

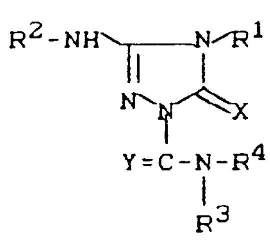

(I)

in welcher

$R^1$, $R^2$, $R^3$, $R^4$, X und Y die oben angegebene Bedeutung haben,

erhält, wenn man

a) 1-Chlor-(thio)carbonyltriazolone der Formel (II),

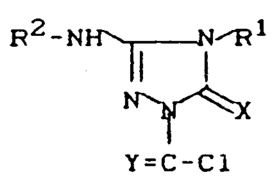

(II)

in welcher
$R^1$, $R^2$, X und Y die oben angegebene Bedeutung haben,
mit Aminen der Formel (III),

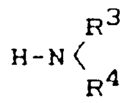

(III)

in welcher
$R^3$ und $R^4$ die oben angegebene Bedeutung haben,
gegebenenfalls in Gegenwart eines Verdünnungsmittels und gegebenenfalls in Gegenwart eines Säurebindemittels umsetzt, oder
b) für den Fall, daß in Formel (I) $R^3$ für Wasserstoff steht, wenn man in 1-Stellung unsubstituierte Triazolone der Formel (IV),

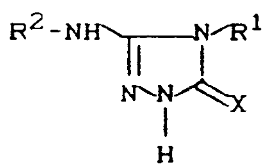

(IV)

in welcher
$R^1$, $R^2$ und X die oben angegebene Bedeutung haben,
mit Iso(thio)cyanaten der Formel (V)

$$R^4\text{-N=C=Y} \qquad (V)$$

in welcher
$R^4$ und Y die oben angegebene Bedeutung haben,
gegebenenfalls in Gegenwart eines Verdünnungsmittels und gegebenenfalls in Gegenwart eines Reaktionshilfsmittels umsetzt.

Schließlich wurde gefunden, daß die neuen substituierten Triazolone der allgemeinen Formel (I) herbizide Eigenschaften besitzen.

Überraschenderweise zeigen die erfindungsgemäßen substituierten Triazolone der allgemeinen Formel (I) eine erheblich höhere herbizide Potenz gegenüber Problemunkräutern als die aus dem Stand der Technik bekannten substituierten Triazolone, wie beispielsweise die Verbindung 1-(Cyclohexylaminocarbonyl)-3-dimethylamino-4-methyl-1,2,4-triazolin-5-on, welche chemisch und wirkungsmäßig naheliegende Verbindungen sind.

Die erfindungsgemäßen substituierten Triazolone sind durch die Formel (I) allgemein definiert. Bevorzugt sind Verbindungen der Formel (I), bei welchen

$R^1$ für Wasserstoff, Methyl, Ethyl, n- oder i-Propyl, n-, i-, s- oder t-Butyl, n- oder i-Pentyl, n- oder i-Hexyl, für Allyl, Propargyl, Methoxy, Ethoxy, Methoxymethyl, für geradkettiges oder verzweigtes Halogenalkyl mit 1 bis 4 Kohlenstoffatomen und 1 bis 9 gleichen oder verschiedenen Halogenatomen, insbesondere Fluor, Chlor oder Brom, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden substituiertes Benzyl oder Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^2$ für Methyl, Ethyl, n- oder i-Propyl, n-, i-, s- oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Methoxymethyl, Methoxyethyl, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden substituiertes Benzyl, Phenylethyl oder Phenyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^3$ und $R^4$ unabhängig voneinander jeweils für Wasserstoff, Methyl, Ethyl, n- oder i-Propyl, n-, i-, s- oder t-Butyl, n- oder i-Pentyl, n -oder i-Hexyl, n- oder i-Heptyl, n- oder i-Octyl, n- oder i-Nonyl, n- oder i-Decyl, n- oder i-Dodecyl, für Allyl, n- oder i-Butenyl, n- oder i-Pentenyl, n- oder i-Hexenyl, Propargyl, n-oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 8 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen, für jeweils gegebenenfalls geradkettiges oder verzweigtes Methoximinoalkyl oder Ethoximinoalkyl mit jeweils 1 bis 5 Kohlenstoffatomen im Alkylteil, oder für jeweils gegebenenfalls ein- bis dreifach, gleich oder - verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl stehen, wobei als Substituenten jeweils infrage kommen: Fluor, Chlor, Brom, Methyl, Ethyl, n- oder i-Propyl, n-, i-, s-oder t-Butyl, Cyano, Methandiyl, Ethandiyl, Butandiyl oder Butadiendiyl; $R^3$ und $R^4$ außerdem unabhängig voneinander für im Heterocyclylteil gegebenenfalls ein- bis dreifach, gleich oder verschieden substituiertes Heterocyclylmethyl, Heterocyclylpropyl, Heterocyclylethyl oder Heterocyclylbutyl stehen, wobei als Heterocyclen jeweils infrage kommen:

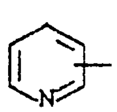 ; 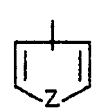 ; 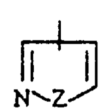 ; 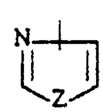 ;

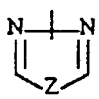 ; 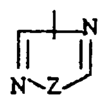 ; 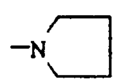 ; 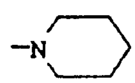 ;

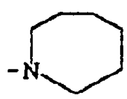 ; 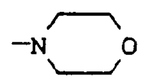 oder 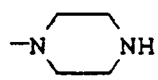

wobei Z jeweils für Sauerstoff oder Schwefel steht und wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio; $R^3$ und $R^4$ außerdem unabhängig voneinander für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein-bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl stehen, wobei als Phenylsubstituenten jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trilfuormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy; oder

$R^3$ und $R^4$ gemeinsam mit dem Stickstoffatom, an welches sie gebunden sind, für einen gegebenenfalls ein- bis dreifach, gleich oder verschieden substituierten Heterocyclus der Formel

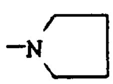 ; 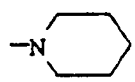 ; 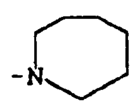 ; 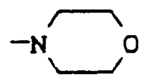 ;

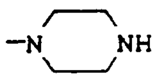 ; 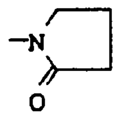 ; 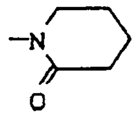 ; 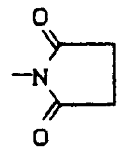 ;

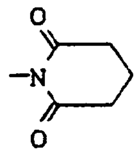 ; 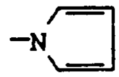 oder 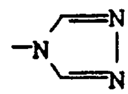

stehen

wobei als Substituenten infrage kommen: Methyl, Ethyl, n- oder i-Propyl, Chlor oder Trifluormethyl,

X für Sauerstoff oder Schwefel steht und

Y für Sauerstoff oder Schwefel steht.

Verwendet man beispielsweise 1-Chlorcarbonyl-3-methylamino-4-methyl-1,2,4-triazolin-5-on und Allylamin als Ausgangsstoffe, so läßt sich der Reaktionsablauf des erfindungsgemäßen Verfahrens (a) durch das folgende Formelschema darstellen:

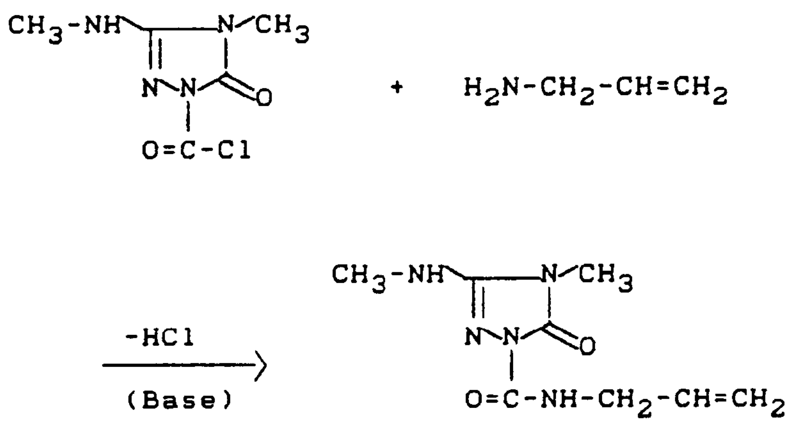


Verwendet man beispielsweise 3-Ethylamino-4-methyl-1H-1,2,4-triazolin-5-on und Isopropylisocyanat als Ausgangsstoffe, so läßt sich der Reaktionsablauf des erfindungsgemäßen Verfahrens (b) durch das folgende Formelschema darstellen:

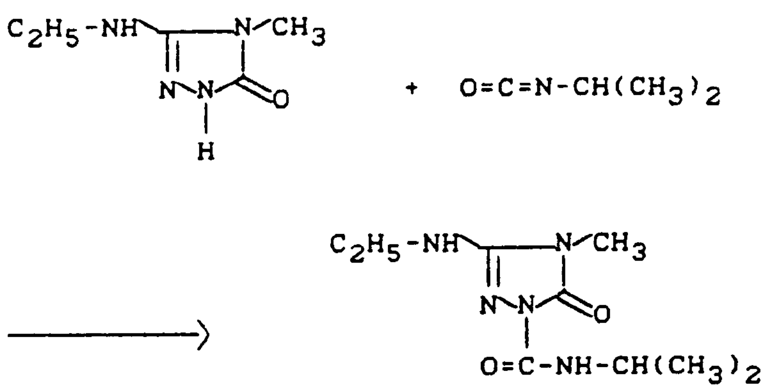

Die zur Durchführung des erfindungsgemäßen Verfahrens (a) als Ausgangsstoffe benötigten Chlor(thio)carbonyltriazolone sind durch die Formel (II) allgemein definiert. In dieser Formel (II) stehen $R^1$, $R^2$, X und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Chlor(thio)carbonyltriazolone der Formel (II) sind noch nicht bekannt.

Man erhält sie, wenn man in 1-Stellung unsubstituierte Triazolone der Formel (IV),

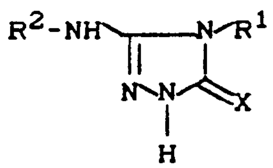

(IV)

in welcher
$R^1$, $R^2$ und X die oben angegebene Bedeutung haben,
mit (Thio)Phosgen der Formel (VI),

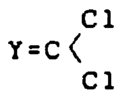

(VI)

in welcher
Y die oben angegebene Bedeutung hat,
gegebenenfalls in Gegenwart eines Verdünnungsmittels wie beispielsweise Toluol, Chlorbenzol oder Acetonitril und gegebenenfalls in Gegenwart eines Säurebindemittels wie beispielsweise Triethylamin bei Temperaturen zwischen + 20° C und + 150° C umsetzt.

Die zur Durchführung des erfindungsgemäßen Verfahrens (a) weiterhin als Ausgangsstoffe benötigten Amine sind durch die Formel (III) allgemein definiert. In dieser Formel (III) stehen $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die Amine der Formel (III) sind allgemein bekannte Verbindungen der organischen Chemie.

Die zur Durchführung des erfindungsgemäßen Verfahrens (b) und zur Synthese der Vorprodukte der Formel (II) als Ausgangsstoffe benötigten in 1-Stellung unsubstituierten Triazolone sind durch die Formel (IV) allgemein definiert. In dieser Formel (IV) stehen $R^1$, $R^2$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die in 1-Stellung unsubstituierten Triazolone der Formel (IV) sind bekannt oder erhältlich in Analogie zu bekannten Verfahren (vgl. z.B. EP 283876; Arch. Pharm. 303, 263-267 [1970]; Arch. Pharm. 307, 509-516 [1974]; J.Org.Chem. 34, 1808-1816 [1969]; Chem. Ber. 107, 454-459 [1974]; J. Heterocycl. Chem. 15, 377-384 [1978]; Pharmazie 29, 20-25 [1974]; Indian J. Chem. 8, 391-394 [1970]; Indian J. Chem. 6, 287-293 [1968]; JP 46/37646; JP 62/2248; JP 62/153850; DE 2714880; DE 2716707; Indian J.Chem.Sect.B 21B, 321-324 [1982]; Helv. Chim. Acta 63, 841-859 [1980]; DE 2145414; J.Chem.Soc.C 1967, 2471-2472; J.Chem.Soc.C 1967, 746-751; J.Chem.Soc.C 1968, 1375-1380; J.Chem.Soc.C 1967, 742-746; GB 1049111; Arch. Pharm. 306, 659-664 [1973]; Farmacia 15, 415-419 [1967] sowie die Herstellungsbeispiele).

Neu sind die Verbindungen der Formel (IV), in welcher

(α) X für Sauerstoff steht und $R^1$ und $R^2$ für Methyl stehen, oder in welcher

(β) X für Sauerstoff oder Schwefel steht und $R^1$ für Methyl und gleichzeitig $R^2$ für Ethyl steht oder $R^1$ für Ethyl und gleichzeitig $R^2$ für Methyl steht oder $R^1$ und $R^2$ für Ethyl stehen, ausgenommen die Verbindung 4-Ethyl-5-(ethylamino-2,4-dihydro-1,2,4-triazol-3-thion (siehe Chemicals Abstracts, 9th Coll Formula Index, (1972-1976) Seite 1831 f).

Man erhält die neuen und bekannten Verbindungen der Formel (IV) beispielsweise, wenn man Aminoguanidiniumhydrochloride (VIIb),

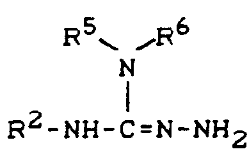

xHCl (VIIb)

in welcher

$R^5$ und $R^6$ für Alkyl, insbesondere für Methyl oder Ethyl stehen und
$R^2$ die oben angegebene Bedeutung hat,
mit Iso(thio)cyanaten der Formel (X),

$$R^1\text{-N=C=X} \qquad (X)$$

in welcher
$R^1$ und X die oben angegebene Bedeutung haben,
gegebenenfalls in Gegenwart eines Verdünnungsmittels wie beispielsweise Toluol, Chlorbenzol oder Acetonitril und gegebenenfalls in Gegenwart eines Säurebindemittels wie beispielsweise Kaliumcarbonat oder Triethylamin bei Temperaturen zwischen 20 und 150° C umsetzt oder wenn man 3-Alkylthiotriazolone der Formel (XI),

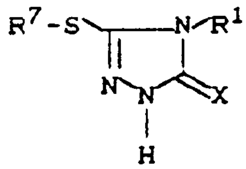

(XI)

in welcher
$R^7$ für Alkyl, insbesondere für Methyl oder Ethyl steht und
$R^1$ und X die oben angegebene Bedeutung haben,
mit Aminen der Formel (XII),

$$R^2\text{-NH}_2 \qquad (XII)$$

in welcher
$R^2$ die oben angegebene Bedeutung hat,
gegebenenfalls in Gegenwart eines Verdünnungsmittels wie beispielsweise Ethanol oder Acetonitril bei Temperaturen zwischen 50° und 300° C in Druckbereichen zwischen Normaldruck und 300 bar umsetzt.

Aminoguanidiniumhydrochloride der Formel (VIIb) sind bekannt oder erhältlich in Analogie zu bekannten Verfahren (vgl. z.B. EP 283876; J.Org.Chem. 19, 1807 [1954]; Bull.Soc.Chim. France 1975, 1649; US-PS 2.845.458).

Iso(thio)cyanate der Formel (X) sind allgemein bekannte Verbindungen der organischen Chemie.

3-Alkylthiotriazolone der Formel (XI) sind ebenfalls bekannt (vgl. z.B. EP 283876; DE 2527676; DE 2250572; US 4.098.896; US 4.110.332; US 4.530.898; J.Chem.Soc.C 1967, 746-751).

Amine der Formel (XII) sind allgemein bekannte Verbindungen der organischen Chemie.

Die zur Durchführung des erfindungsgemäßen Verfahrens (b) weiterhin als Ausgangsstoffe benötigten Iso(thio)cyanate sind durch die Formel (V) allgemein definiert. In dieser Formel (V) stehen $R^4$ und Y vorzugsweise für diejenigen Reste, die bereits im Zusammenhang mit der Beschreibung der erfindungsgemäßen Stoffe der Formel (I) als bevorzugt für diese Substituenten genannt wurden.

Die Iso(thio)cyanate sind allgemein bekannte Verbindungen der organischen Chemie (vgl. z.B. Saul Patai, "The Chemistry of Cyanates and their Thioderivates" J. Wiley & Sons, New York 1977).

Als Verdünnungsmittel zur Durchführung des erfindungsgemäßen Verfahrens (a) kommen vorzugsweise inerte organische Lösungsmittel infrage.

Hierzu gehören insbesondere aliphatische, alicyclische oder aromatische, gegebenenfalls halogenierte Kohlenwasserstoffe, wie beispielsweise Benzin, Ligroin, Benzol, Toluol, Xylol, Chlorbenzol, Petrolether, Pentan, Hexan, Heptan, Cyclohexan, Dichlormethan, Chloroform, Tetrachlorkohlenstoff, Ether, wie Diethylether, Dioxan, Tetrahydrofuran oder Ethylenglykoldimethyl oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Basen wie Pyridin.

Das erfindungsgemäße Verfahren (a) wird gegebenenfalls in Gegenwart eines geeigneten Säurebindemittels durchgeführt.

Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist auch möglich, das als Reaktionspartner verwendete Amin der Formel (III) in entsprechendem Überschuß gleichzeitig als Säurebindemittel einzusetzen.

Die Reaktionstemperaturen können bei der Durchführung des erfindungsgemäßen Verfahrens (a) in einem größeren Bereich variiert werden. Im allgemeinen arbeitet man bei Temperaturen zwischen 0° C und + 150° C, vorzugsweise bei Temperaturen zwischen + 10° C und + 80° C.

Das erfindungsgemäße Verfahren (a) wird üblicherweise unter Normaldruck durchgeführt. Es ist jedoch auch möglich unter erhöhtem Druck zu arbeiten.

Zur Durchführung des erfindungsgemäßen Verfahrens (a) setzt man pro Mol an 1-Chlor-(thio)carbonyltriazolon der Formel (II) im allgemeinen 1,0 bis 5,0 Mol, vorzugsweise 1,0 bis 2,5 Mol, am Amin der Formel (III) und gegebenenfalls 1,0 bis 2,5 Mol an Säurebindemittel ein. Die Reaktionsdurchführung, Aufarbeitung und Isolierung der Reaktionsprodukte erfolgt in Analogie zu allgemein bekannten Verfahren.

Als Verdünnungmittel zur Durchführung des erfindungsgemäßen Verfahrens (b) kommen ebenfalls inerte organische Lösungsmittel infrage. Vorzugsweise verwendet man die bei Verfahren (a) genannten Verdünnungsmittel.

Das erfindungsgemäße Verfahren (b) kann gegebenenfalls in Gegenwart eines basischen Reaktionshilfsmittels durchgeführt werden. Als solche kommen alle üblichen anorganischen und organischen Basen infrage. Vorzugsweise verwendet man tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Der Zusatz solcher Katalysatoren ist jedoch nicht zwingend erforderlich.

Die Reaktionstemperaturen können bei der Durchführung des erfindungsgemäßen Verfahrens (b) in einem größeren Bereich variiert werden. Im allgemeinen arbeitet man bei Temperaturen zwischen 0° C und + 150° C, vorzugsweise bei Temperaturen zwischen + 40° C und +120 ° C.

Das erfindungsgemäße Verfahren (b) wird üblicherweise unter Normaldruck durchgeführt. Es ist jedoch auch möglich, insbesondere bei gasförmigen Ausgangsverbindungen, unter erhöhtem Druck zu arbeiten.

Zur Durchführung des erfindungsgemäßen Verfahrens (b) setzt man pro Mol an in 1-Stellung unsubstituiertem Triazolon der Formel (IV) im allgemeinen 1,0 bis 5,0 Mol, vorzugsweise 1,0 bis 2,5 Mol, an Iso(thio)cyanat der Formel (V) und gegebenenfalls 1,0 bis 2,5 Mol an Reaktionshilfsmittel ein. Die Reaktionsdurchführung, Aufarbeitung und Isolierung der Reaktionsprodukte erfolgt in Analogie zu allgemein bekannten Verfahren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u> Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u> Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u> Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur Bekämpfung dikotyler Unkräuter insbesondere in monokotylen Kulturen wie beispielsweise Mais einsetzen.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter

Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

In entsprechenden Aufwandmengen zeigen die erfindungsgemäßen Wirkstoffe außerdem auch fungizide Wirksamkeit und lassen sich beispielsweise zur Bekämpfung von Pilzkrankheiten im Getreide- und Reisanbau, wie beispielsweise gegen den Erreger des Getreidemehltaus (Erysiphe graminis) oder gegen den Erreger der Reisfleckenkrankheit (Pyricularia oryzae) oder zur Bekämpfung von Pilzkrankheiten im Obst- und Gemüseanbau, wie beispielsweise gegen den Erreger des Apfelschorfes (Venturia inaequalis) oder gegen Cercospora-Arten einsetzen. In diesem Anwendungsbereich zeigen die erfindungsgemäßen Wirkstoffe neben guten protektiven auch systemische Eigenschaften.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)N,N'-dimethyl-harnstoff (METABENZTHIAZURON) zur Unkrautbekämpfung in Getreide; 4-Amino-3-methyl-6-phenyl-1,2,4-triazin-5(4H)-on zur Unkrautbekämpfung (METAMITRON) in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}- benzoesäure oder deren Methylester (METSULFURON); N-(1-Ethylpropyl)-3,4-dimethyl-2,6-dinitroanilin (PENDIMETHALIN); 0-(6-Chlor-3-phenylpyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]- thiophen-2-carbonsäure-methylester (THIAMETURON); N,N-Diisopropyl-S-(2,3,3-trichlorallyl)-thiolcarbamat (TRIALLATE) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Herstellungsbeispiele:

Beispiel 1:

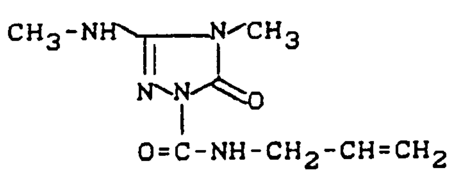

(Verfahren b)

Zu 6.4 g (0.05 Mol) 3-Methylamino-4-methyl-(1H)-1,2,4-triazolin-5-on in 150 ml Acetonitril gibt man 4.2 g (0.05 Mol) Allylisocyanat und 3 Tropfen Diazabicycloundecen (DBU), wobei die Temperatur der Reaktionsmischung auf 30° C ansteigt. Anschließend rührt man 30 Minuten bei Rückflußtemperatur, engt dann im Vakuum ein und kristallisiert aus Essigester um.

Man erhält 7.9 g (75 % der Theorie) an 1-Allylaminocarbonyl-3-methylamino-4-methyl-1,2,4-triazolin-5-on vom Schmelzpunkt 136-137° C.

Beispiel 2:

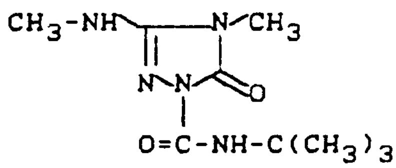

(Verfahren b)

Zu 6.4 g (0.05 Mol) 3-Methylamino-4-methyl-(1H)-1,2,4-triazolin-5-on in 150 ml Acetonitril gibt man 4.95 g (0.05 Mol) t-Butylisocyanat und 3 Tropfen Diazabicycloundecen (DBU), rührt anschließend 30 Minuten bei Rückflußtemperatur, filtriert und engt das Filtrat im Vakuum ein.

Man erhält 6.5 g (57 % der Theorie) an 1-t-Butylaminocarbonyl-3-methylamino-4-methyl-1,2,4-triazolin-5-on vom Schmelzpunkt 210° C (Zersetzung).

Beispiel 3:

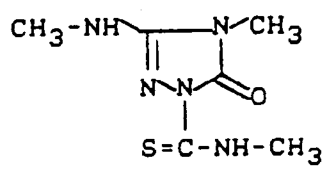

(Verfahren b)

Zu 6.4 g (0.05 Mol) 3-Methylamino-4-methyl-(1H)-1,2,4-triazolin-5-on in 150 ml Acetonitril gibt man 3.65 g (0.05 Mol) Methylisothiocyanat und 3 Tropfen Diazabicycloundecen (DBU), rührt anschließend 30 Minuten bei 40° C bis 50° C und danach weitere 30 Minuten bei 70° C bis 80° C, engt dann im Vakuum ein und kristallisiert aus Essigester/Acetonitril (3:1) um.

Man erhält 4.95 g (49 % der Theorie) an 1-Methylaminothiocarbonyl-3-methylamino-4-methyl-1,2,4-triazolin-5-on vom Schmelzpunkt 161-163° C.

Beispiel 4:

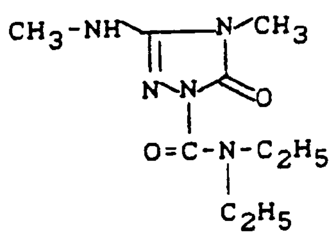

(Verfahren a)

Zu 11.4 g (0.06 Mol) 1-Chlorcarbonyl-3-methylamino-4-methyl-(1,2,4)-triazolin-5-on in 200 ml Acetonitril gibt man unter Rühren tropfenweise 8.83 g (0.12 Mol) Diethylamin, so daß die Temperatur der Reaktionsmischung 40° C nicht übersteigt. Nach beendeter Zugabe rührt man weitere 2 Stunden bei Raumtemperatur, filtriert, engt das Filtrat im Vakuum ein, nimmt den Rückstand in 150 ml Aceton auf, filtriert erneut und engt das Filtrat wiederum im Vakuum ein.

Man erhält 12.2 g (90 % der Theorie) an 1-Diethylaminocarbonyl-3-methylamino-4-methyl-1,2,4-triazolin-5-on vom Schmelzpunkt 83° C bis 85° C.

Beispiel 5:

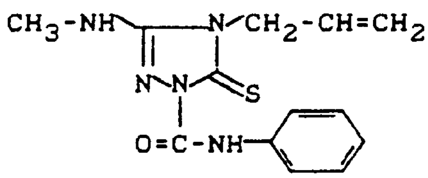

(Verfahren b)

Zu 8.5 g (0.05 Mol) 3-Methylamino-4-allyl-(1H)-1,2,4-triazolin-5-thion in 150 ml Acetonitril gibt man 5.95 g (0.05 Mol) Phenylisocyanat, rührt anschließend 1 Stunde bei Rückflußtemparatur, engt dann im Vakuum ein und kristallisiert den Rückstand aus Toluol/Cyclohexan (1:1) um.

Man erhält 8.7 g (60 % der Theorie) an 1-Phenylaminocarbonyl-3-methylamino-4-allyl-1,2,4-triazolin-5-thion vom Schmelzpunkt 132°-133° C.

In entsprechender Weise und gemäß den allgemeinen Angaben zur Herstellung erhält man die folgenden substituierten Triazolone der allgemeinen Formel (I):

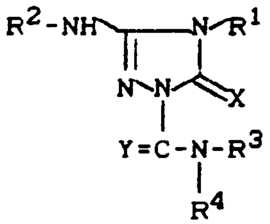

(I)

Tabelle 1

| Bsp. Nr. | $R^1$ | $R^2$ | -N-$R^3$<br>$R^4$ | X | Y | Schmelzpunkt/°C |
|---|---|---|---|---|---|---|
| 6 | $CH_3$ | $CH_3$ | $-NH-CH_2-C(CH_3)_3$ | O | O | 208-209 |
| 7 | $CH_3$ | $CH_3$ | -NH- 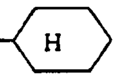 | O | O | 172-173 |
| 8 | $CH_3$ | $CH_3$ | $-NH-CH_3$ | O | O | 226-227 |
| 9 | $CH_3$ | $CH_3$ | $-NH-C_2H_5$ | O | O | 182-183 |
| 10 | $CH_3$ | $CH_3$ | $-NH-(CH_2)_2-CH_3$ | O | O | 124-125 |
| 11 | $CH_3$ | $CH_3$ | $-NH-CH(CH_3)_2$ | O | O | 160-162 |
| 12 | $CH_3$ | $CH_3$ | $CH_3$<br>$-NH-C-CH_2Cl$<br>$CH_3$ | O | O | 221 |
| 13 | $CH_2=CH-CH_2-$ | $CH_3$ | $-NH-CH_2-C(CH_3)_3$ | O | O | 182-183 |
| 14 | $CH_2=CH-CH_2-$ | $CH_3$ | -NH- 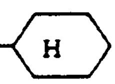 | S | O | 147 |
| 15 | $CH_3$ | $CH_3$ | -NH- 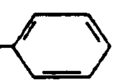 | O | O | 104 |
| 16 | $CH_3$ | $CH_3$ | $-NH-CH_2-CH=CH_2$ | O | S | 161-162 |
| 17 | $CH_2=CH-CH_2-$ | $CH_3$ | $-NH-CH_3$ | S | O | 144 |
| 18 | $CH_2=CH-CH_2-$ | $CH_3$ | $-NH-(CH_2)_2-CH_3$ | S | O | 117 |

T a b e l l e  1 - Fortsetzung

| Bsp. Nr. | $R^1$ | $R^2$ | $-N(R^4)-R^3$ | X | Y | Schmelz-punkt/°C |
|---|---|---|---|---|---|---|
| 19 | $CH_2=CH-CH_2-$ | $CH_3$ | $-NH-CH(CH_3)_2$ | S | O | 126-127 |
| 20 | $CH_3$ | $CH_3$ | $-NH-(CH_2)_3-CH_3$ | O | O | 110 |
| 21 | $CH_3$ | $CH_3$ | $-NH-CH_2-C(CH_3)_3$ | S | O | 203 |
| 22 | $CH_3$ | $CH_3$ | -NH 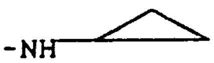 | O | O | 191 |
| 23 | $CH_3$ | $CH_3$ | -NH 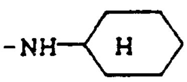 | S | O | 180-181 |
| 24 | $CH_3$ | $CH_3$ | -NH 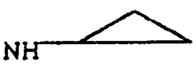 | S | O | 187 |
| 25 | $CH_3$ | $CH_3$ | $-NH-(CH_2)_2-CH_3$ | S | O | 157-159 |
| 26 | $CH_3$ | $CH_3$ | $CH_3$<br>\|<br>$-NH-C-CH_2Cl$<br>\|<br>$CH_3$ | S | O | 196 |
| 27 | $CH_3$ | $CH_3$ | $CH_2Cl$<br>\|<br>$-NH-C-CH_3$<br>\|<br>$CH_2Cl$ | S | O | 183-185 |
| 28 | $CH_3$ | $CH_3$ | -NH 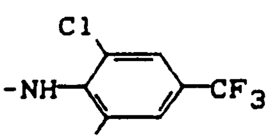 | O | O | 231-232 |
| 29 | $CH_3$ | $CH_3$ | $CH_3$<br>\|<br>$-NH-CH-C_2H_5$ | O | O | 181-182 |

T a b e l l e 1 - Fortsetzung

| Bsp. Nr. | $R^1$ | $R^2$ | $-N-R^3$<br>$R^4$ | X | Y | Schmelzpunkt/° C |
|---|---|---|---|---|---|---|
| 30 | $CH_3$ | $CH_3$ | $-NH-CH_2-CH_2Cl$ | O | O | 215 |
| 31 | $CH_3$ | $CH_3$ | $-NH-CH_2-COOC_2H_5$ | O | O | 163-164 |
| 32 | $CH_3$ | $CH_3$ | 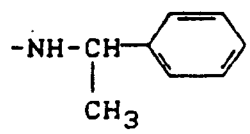 | O | O | 165 |
| 33 | $CH_3$ | $CH_3$ | 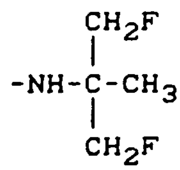 | O | O | 196-197 |
| 34 | $CH_3$ | $CH_3$ | 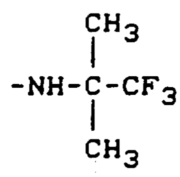 | O | O | 259-262 |
| 35 | $CH_3$ | $CH_3$ | 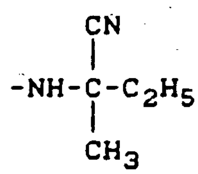 | O | O | 222-224 |
| 36 | $CH_3$ | $CH_3$ | 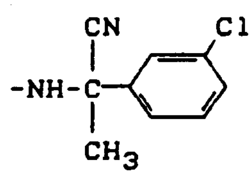 | O | O | 165 |
| 37 | $CH_3$ | $CH_3$ | $-NH-CH_2-CH_2-OC_2H_5$ | O | O | 115 |

Tabelle 1 - Fortsetzung

| Bsp. Nr. | $R^1$ | $R^2$ | $-N-R^3$<br>$R^4$ | X | Y | Schmelzpunkt /° C |
|---|---|---|---|---|---|---|
| 38 | $CH_3$ | $CH_3$ | 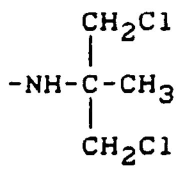 | O | O | 215 |
| 39 | $CH_3$ | $CH_3$ | 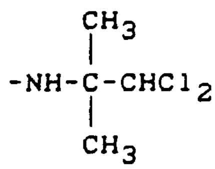 | O | O | 216 |
| 40 | $CH_3$ | $CH_3$ | 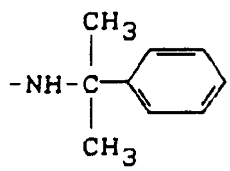 | O | O | 169-170 |
| 41 | $CH_3$ | $CH_3$ | 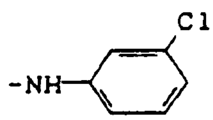 | O | O | 221-224 |
| 42 | $CH_3$ | $CH_3$ | 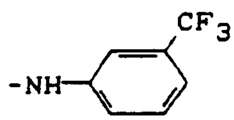 | O | O | 180-182 |
| 43 | $C_2H_5$ | $CH_3$ | 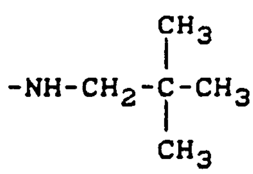 | O | O | 181-182 |
| 44 | $C_2H_5$ | $CH_3$ | 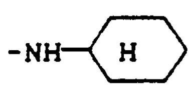 | O | O | 145-146 |
| 45 | $C_2H_5$ | $CH_3$ | 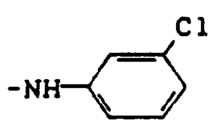 | O | O | 160-162 |

Tabelle 1 - Fortsetzung

| Bsp. Nr. | $R^1$ | $R^2$ | $-N-R^3$<br>\|<br>$R^4$ | X | Y | Schmelzpunkt /°C |
|---|---|---|---|---|---|---|
| 46 | $C_2H_5$ | $CH_3$ | $-NH-CH(CH_3)_2$ | O | O | 144-146 |
| 47 | $CH_3$ | $CH_3$ | 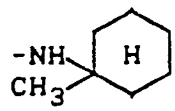 | O | O | 238-239 |
| 48 | $CH_3$ | $CH_3$ | 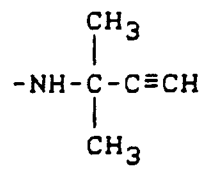 | O | O | 200 (Zers.) |
| 49 | $CH_3$ | $CH_3$ | 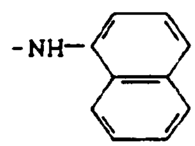 | O | O | 208-211 |
| 50 | $CH_3$ | $CH_3$ | 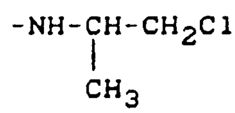 | O | O | 155-156 |
| 51 | $CH_3$ | $CH_3$ | 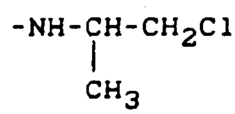 | S | O | 180 |
| 52 | $CH_3$ | $CH_3$ | 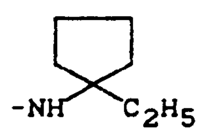 | O | O | 233-234 |
| 53 | $CH_3$ | $CH_3$ | 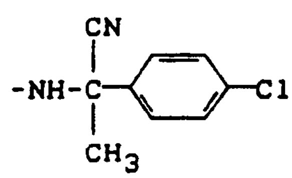 | O | O | 220-221 |

T a b e l l e  1 - Fortsetzung

| Bsp. Nr. | $R^1$ | $R^2$ | $-N(R^3)R^4$ | X | Y | Schmelzpunkt/° C |
|---|---|---|---|---|---|---|
| 54 | $CH_3$ | $CH_3$ | 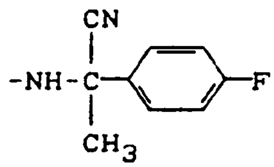 | O | O | 208 (Zers.) |
| 55 | $CH_3$ | $CH_3$ | 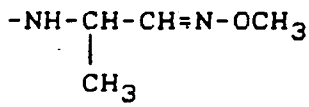 | O | O | 117-119 |
| 56 | $CH_3$ | $CH_3$ | $-NH-C_2H_5$ | O | S | 150-151 |
| 57 | $CH_3$ | $CH_3$ | $-NH-(CH_2)_5-CH_2Cl$ | O | O | 106 |
| 58 | $CH_3$ | $CH_3$ | 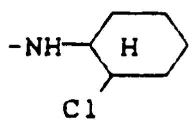 | O | O | 219-220 |
| 59 | $CH_3$ | $CH_3$ | 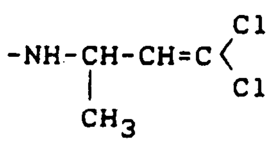 | O | O | 174-176 |
| 60 | $CH_3$ | $CH_3$ | 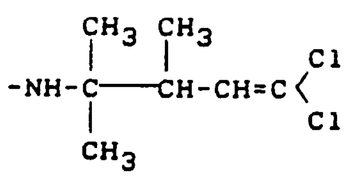 | O | O | 195-196 |
| 61 | $CH_3$ | $CH_3$ | 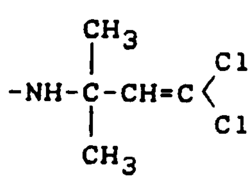 | O | O | 211-213 |

T a b e l l e 1 - Fortsetzung

| Bsp. Nr. | $R^1$ | $R^2$ | $-N-R^3$<br>$R^4$ | X | Y | Schmelzpunkt/° C |
|---|---|---|---|---|---|---|
| 62 | $CH_3$ | $CH_3$ | $CH_2Cl$<br>$-NH-CH$ / $-NH-CH_2-CH-Cl$<br>$CH_2Cl$ $CH_2-Cl$<br>(60 : 40) | O | O | 162-163 |
| 63 | $CH_3$ | $CH_3$ | $CH_3$<br>$-C-C_2H_5$<br>$CH_3$ | O | O | 221-222 |
| 64 | $CH_3$ | $CH_3$ | $-NH-(CH_2)_5-CH_3$ | O | O | 100-102 |
| 65 | $CH_3$ | $CH_3$ | $-NH-CH_2-CH(CH_3)_2$ | O | O | 155-157 |
| 66 | $CH_3$ | $CH_3$ | $-NH-CH_2-CH-(CH_2)_3-CH_3$<br>$C_2H_5$ | O | O | 140-141 |
| 67 | $CH_3$ | $CH_3$ | $-NH-CH-(CH_2)_2-$ 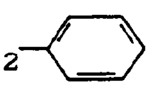<br>$CH_3$ | O | O | 116-118 |
| 68 | $CH_3$ | $CH_3$ | $-NH-OCH_3$ | O | O | 167-170 |
| 69 | $CH_3$ | $CH_3$ | $-NH-CH_2-$ 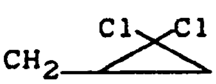 | O | O | 182-183 |
| 70 | $CH_3$ | $CH_3$ | $-NH-CH-CH_2-CH(CH_3)_2$<br>$CH_3$ | O | O | 170-173 |

Tabelle 1 - Fortsetzung

| Bsp. Nr. | $R^1$ | $R^2$ | $-N(R^4)-R^3$ | X | Y | Schmelzpunkt/°C |
|---|---|---|---|---|---|---|
| 71 | $CH_3$ | $CH_3$ | $-NH-CH_2-CH_2-CH(CH_3)_2$ | O | O | 116-120 |
| 72 | $CH_3$ | $CH_3$ | 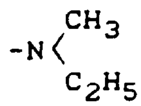 | O | O | 109-111 |
| 73 | $CH_3$ | $CH_3$ | 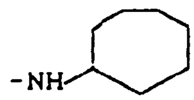 | O | O | 203-206 |
| 74 | $CH_3$ | $CH_3$ | 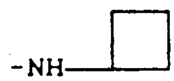 | O | O | 173-176 |
| 75 | $CH_3$ | $CH_3$ | 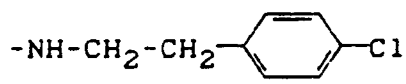 | O | O | 173-175 |
| 76 | $CH_3$ | $CH_3$ | 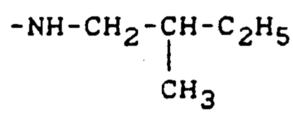 | O | O | 156-158 |
| 77 | $CH_3$ | $CH_3$ | 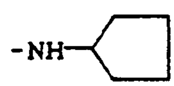 | O | O | 187-189 |
| 78 | $CH_3$ | $CH_3$ | 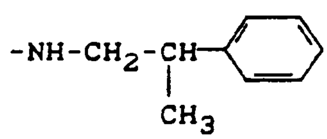 | O | O | 63- 65 |
| 79 | $CH_3$ | $CH_3$ | 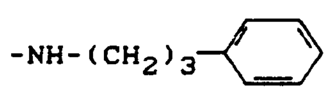 | O | O | 79- 81 |

T a b e l l e 1 - Fortsetzung

| Bsp. Nr. | $R^1$ | $R^2$ | $-N-R^3$<br>\|<br>$R^4$ | X | Y | Schmelzpunkt/°C |
|---|---|---|---|---|---|---|
| 80 | $CH_3$ | $CH_3$ | $-NH-(CH_2)_4-$ 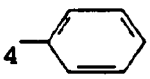 | O | O | 107-110 |
| 81 | $CH_3$ | $CH_3$ | $-NH-CH-(CH_2)_4-CH_3$<br>\|<br>$CH_3$ | O | O | 127-129 |
| 82 | $CH_3$ | $CH_3$ | $-NH-CH-(CH_2)_2-CH_3$<br>\|<br>$CH_3$ | O | O | 160-163 |
| 83 | $CH_3$ | $CH_3$ | $-NH-CH-CH(CH_3)_2$<br>\|<br>$CH_3$ | O | O | 222-223 |
| 84 | $CH_3$ | $CH_3$ | $-NH-CH-(CH_2)_3-CH(CH_3)_2$<br>\|<br>$CH_3$ | O | O | 159-161 |
| 85 | $CH_3$ | $CH_3$ | $-NH-CH-(CH_2)_3-CH_3$<br>\|<br>$C_2H_5$ | O | O | 150-151 |
| 86 | $CH_3$ | $CH_3$ | $-NH-CH-CH_2-OCH_3$<br>\|<br>$CH_3$ | O | O | 139-141 |
| 87 | $CH_3$ | $CH_3$ | $C_2H_5$<br>\|<br>$-NH-C-CH_3$<br>\|<br>$C_2H_5$ | O | O | 226 |

Tabelle 1 - Fortsetzung

| Bsp. Nr. | $R^1$ | $R^2$ | 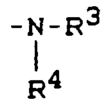 | X | Y | Schmelzpunkt /° C |
|---|---|---|---|---|---|---|
| 88 | $CH_3$ | $CH_3$ | $-NH-C(C_2H_5)_3$ | O | O | 208-210 |
| 89 | $CH_3$ | $CH_3$ | 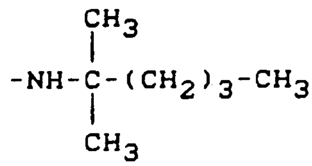 | O | O | 186-187 |
| 90 | $CH_3$ | $CH_3$ | 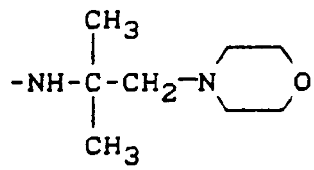 | O | O | 159-161 |
| 91 | $CH_3$ | $CH_3$ | 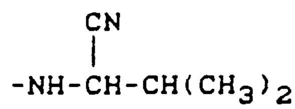 | O | O | 134-136 |
| 92 | $CH_3$ | $CH_3$ | 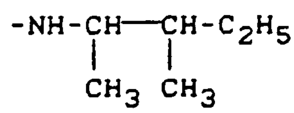 | O | O | 194-196 |
| 93 | $CH_3$ | $C_2H_5$ | $-NH-CH_3$ | O | O | 215-217 |
| 94 | $CH_3$ | $C_2H_5$ | $-NH-CH(CH_3)_2$ | O | O | 148-150 |
| 95 | $CH_3$ | $C_2H_5$ | 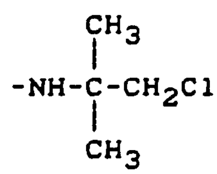 | O | O | 176-178 |
| 96 | $CH_3$ | $C_2H_5$ | 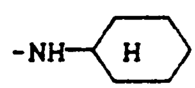 | O | O | 185-187 |

Tabelle 1 - Fortsetzung

| Bsp. Nr. | $R^1$ | $R^2$ | $-\underset{R^4}{N}-R^3$ | X | Y | Schmelzpunkt/°C |
|---|---|---|---|---|---|---|
| 97 | $CH_3$ | $C_2H_5$ | $-NH-\underset{CH_3}{CH}-C_2H_5-$ | O | O | 143-145 |
| 98 | $CH_3$ | $(CH_3)_2CH-$ | $-NH-CH_3$ | O | O | 184-187 |
| 99 | $CH_3$ | $(CH_3)_2CH-$ | $-NH-CH(CH_3)_2$ | O | O | 184-185 |
| 100 | $CH_3$ | $(CH_3)_2CH-$ | $-NH-\underset{CH_3}{CH}-C_2H_5$ | O | O | 189-191 |

Herstellung der Ausgangsprodukte:

Beispiel II-1:

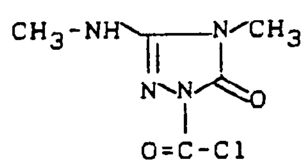

Zu einer gesättigten Lösung von ca. 150 g Phosgen in 150 ml trockenem Chlorbenzol gibt man 12.8 g (0.1 Mol) 3-Methylamino-4-methyl-(1H)-1,2,4-triazolin-5-on und erwärmt unter Rühren und weiterem Einleiten von Phosgen aus Rückflußtemperatur. Nach 30 Minuten treibt man überschüssiges Phosgen mit Hilfe eines trockenen Stickstoffstromes aus der Lösung, saugt ausgefallenes Produkt kalt ab und kristallisiert aus Essigester um.

Man erhält 17.5 g (92 % der Theorie) an 1-Chlorcarbonyl-3-methylamino-4-methyl-1,2,4-triazolin-5-on vom Schmelzpunkt 185° C.

Beispiel IV-1:

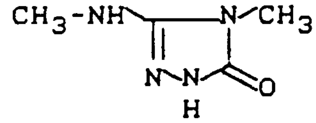

In einem 3000 ml Autoklaven werden 87 g (0.6 Mol) 3-Methylthio-4-methyl-(1H)-1,2,4-triazolin-5-on (vgl. z.B. US 4.098.896 oder US 4.110.332) und 300 g (9.67 Mol) Methylamin in 1000 ml Ethanol unter Rühren 3 Stunden lang auf 230° C erhitzt, wobei sich ein Druck von 75 bar einstellt. Die erkaltete Reaktionsmischung wird eingeengt und der Rückstand aus Acetonitril umkristallisiert.

Man erhält 58 g (76 % der Theorie) an 3-Methyl-amino-4-methyl-(1H)-1,2,4-triazolin-5-on vom Schmelzpunkt 202-204°C.

Beispiel IV-2:

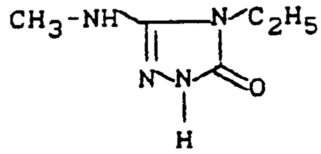

45.75 g (0.3 Mol) 1-Amino-2,2,3-trimethylguanidiniumhydrochlorid (vgl. z.Bsp. EP 283876) und 21.3 g (0.3 Mol) Ethylisocyanat werden in 300 ml Acetonitril 2 Stunden bei Rückflußtemperatur gerührt, anschließend auf 40° C abgekühlt, mit 50.4 g (0.6 Mol) Natriumhydrogencarbonat versetzt und weitere 8 Stunden bei Rückflußtemperatur gerührt. Zur Aufarbeitung wird heiß filtriert und das Filtrat abgekühlt. Das ausgefallene Reaktionsprodukt wird abgesaugt, gewaschen und getrocknet.

Man erhält 25 g (59 % der Theorie) an 3-Methyl-amino-4-ethyl-(1H)-1,2,4-triazolin-5-on vom Schmelzpunkt 208-210°C.

Beispiel IV-3:

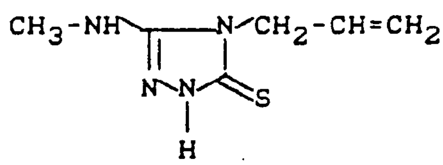

91.5 g (0.6 Mol) 1-Amino-2,2,3-trimethylguanidiniumhydrochlorid und 59.4 g (0.6 Mol) Allylisothiocyanat werden in 800 ml Acetonitril 2 Stunden bei Rückflußtemperatur gerührt, anschließend tropfenweise mit 60.6 g (0.6 Mol) Triethylamin versetzt, eine weitere Stunde bei Rückflußtemperatur gerührt, abgekühlt, filtriert, das Filtrat eingeengt und der ölige Rückstand zwischen Dichlormethan und Wasser verteilt. Die vereinigten organischen Phasen werden über Natriumsulfat getrocknet und eingeengt.

Man erhält 68 g (67 % der Theorie) an 3-Methyl-amino-4-allyl-(1H)-1,2,4-triazolin-5-thion vom Schmelzpunkt 129-131°C.

Beispiel VIIb-1:

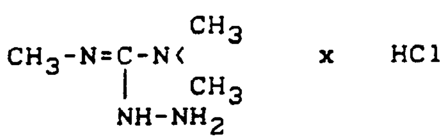

Zu 50 g (1 Mol) Hydrazinhydrat in 300 ml Isopropanol tropft man bei 20° C bis 25° C unter Rühren innerhalb von 30 Minuten eine Lösung von 78,5 g (0,5 Mol) Chlortrimethylformamidiniumhydrochlorid in 250 ml Isopropanol, rührt nach beendeter Zugabe weitere 30 Minuten bei Raumtemperatur, saugt ausgefallenes Hydrazinhydrochlorid ab, wäscht mit 150 ml Isopropanol nach und engt das Isopropanolfiltrat im Vakuum ein.

Man erhält 70,7 g (93 % der Theorie) an 1-Amino-2,2,3-trimethylguanidiniumhydrochlorid, welches ohne Reinigung weiter umgesetzt wird.

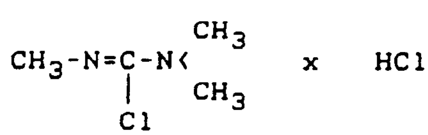

In eine Mischung aus 510 g (5 Mol) N,N,N'-Trimethylharnstoff und 3 Liter Chlorbenzol leitet man bei 80° C innerhalb 2,5 Stunden unter Rühren 545 g (5,5 Mol) Phosgen und rührt nach beendetem Einleiten weitere 45 Minuten bis zum Ende der Kohlendioxid-Entwicklung bei 80° C nach. Die Reaktionsmischung wird abgekühlt

auf 10° C, das wasserempfindliche Produkt unter Stickstoff abgesaugt, mit 1 Liter Chlorbenzol und zweimal mit jeweils 500 ml Petrolether gewaschen und im Vakuum getrocknet.

Man erhält 635,3 g (81 % der Theorie) an Chlortrimethylformamidiniumhydrochlorid vom Schmelzpunkt 76° C bis 78° C.

Anwendungsbeispiele:

In dem folgenden Anwendungsbeispiel wurde die nachstehend aufgeführte Verbindung als Vergleichsubstanz eingesetzt:

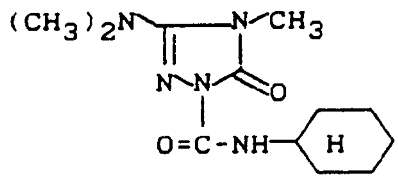

(A)

1-Cyclohexylaminocarbonyl-3-dimethylamino-4-methyl-1,2,4-triazolin-5-on (bekannt aus EP 283876).

Beispiel A:

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5-15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0% = keine Wirkung (wie unbehandelte Kontrolle)
100% = totale Vernichtung

Die Verbindungen gemäß den Herstellungsbeispielen 7, 12, 29, 48, 69 zeigen in diesem Test eine deutliche Überlegenheit in der herbiziden Wirksamkeit bei vergleichbarer Kulturpflanzenselektivität gegenüber der aus dem Stand der Technik bekannten Verbindung (A).

## Patentansprüche

**1.** Substituierte Triazolone der allgemeinen Formel (I),

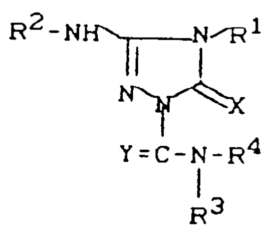

(I)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten infrage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl mit jeweils 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil, Aryl mit 6 bis 10 Kohlenstoffatomen steht, wobei als Substituenten im Arylteil jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^3$ und $R^4$ unabhängig voneinander jeweils für Wasserstoff, für jeweils geradkettiges oder verzweigtes Alkyl mit 1 bis 18 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, für Alkylaminoalkyl, Dialkylaminoalkyl oder Alkoximinoalkyl mit jeweils 1 bis 8 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenene Halogenatomen oder jeweils zweifach verknüpftes Alkandiyl oder Alkendiyl mit jeweils bis zu 4 Kohlenstoffatomen; $R^3$ und $R^4$ außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil stehen, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^3$ und $R^4$ außerdem unabhängig voneinander für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aralkyloxy, Aryloxy, Aroyl oder Aryl mit jeweils 6 bis 10 Kohlenstoffatomen im Arylteil und gegebenenfalls 1 bis 6 Kohlenstoffatomen im Alkylteil stehen, wobei als Arylsubstituenten jeweils infrage kommen: Halogen, Cyano, Nitro,

Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen oder Phenoxy und wobei als Alkylsubstituenten gegebenenfalls infrage kommen: Halogen oder Cyano, oder

$R^3$ und $R^4$ gemeinsam mit dem Stickstoffatom, an welches sie gebunden sind, für einen gegebenenfalls einfach oder mehrfach, gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel, enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen,

X für Sauerstoff oder Schwefel steht und

Y für Sauerstoff oder Schwefel steht.

**2.** Substituierte Triazolone der Formel (I) gemäß Anspruch 1, in welcher

$R^1$ für Wasserstoff, Methyl, Ethyl, n- oder i-Propyl, n-, i-, s- oder t-Butyl, n- oder i-Pentyl, n- oder i-Hexyl, für Allyl, Propargyl, Methoxy, Ethoxy, Methoxymethyl, für geradkettiges oder verzweigtes Halogenalkyl mit 1 bis 4 Kohlenstoffatomen und 1 bis 9 gleichen oder verschiedenen Halogenatomen, für Cyclopentyl, Cyclohexyl, Cyclopropyl, Cyclopropylmethyl, Cyclohexylmethyl, Cyclohexylethyl oder für jeweils gegebenenfalls ein- bis dreifach gleich oder verschieden substituiertes Benzyl oder Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio,

$R^2$ für Methyl, Ethyl, n- oder i-Propyl, n-, i-, s-oder t-Butyl, n- oder i-Pentyl, Allyl, Propargyl, für jeweils geradkettiges oder verzweigtes Halogenalkyl mit 1 bis 4 Kohlenstoffatomen, Halogenalkenyl mit 3 bis 6 Kohlenstoffatomen oder Halogenalkinyl mit 3 bis 6 Kohlenstoffatomen und jeweils 1 bis 9 gleichen oder verschiedenen Halogenatomen, für Methoxymethyl, Methoxyethyl, für Cyclopropyl, Cyclopropylmethyl, Cyclopentyl, Cyclohexyl, Cyclohexylmethyl, Cyclohexylethyl, Cyclopentylmethyl oder für jeweils gegebenenfalls ein- bis dreifach, gleich oder verschieden substituiertes Benzyl, Phenylethyl oder Phenyl steht, wobei als Substituenten jeweils infrage kommen: Fluor, Chlor, Brom, Cyano, Nitro, Methyl, Ethyl, n-oder i-Propyl, n-, i-, s- oder t-Butyl, Methoxy, Ethoxy, Methylthio, Trifluormethyl, Trifluormethoxy oder Trifluormethylthio,

$R^3$ und $R^4$ unabhängig voneinander jeweils für Wasserstoff, Methyl, Ethyl, n- oder i-Propyl, n-, i-, s- oder t-Butyl, n- oder i-Pentyl, n -oder i-Hexyl, n- oder i-Heptyl, n- oder i-Octyl, n- oder i-Nonyl, n- oder i-Decyl, n- oder i-Dodecyl, für Allyl, n-oder i-Butenyl, n- oder i-Pentenyl, n- oder i-Hexenyl, Propargyl, n- oder i-Butinyl, n- oder i-Pentinyl, n- oder i-Hexinyl, für geradkettiges oder verzweigtes Halogenalkyl mit 1 bis 6 Kohlenstoffatomen und 1 bis 9 gleichen oder verschiedenen Halogenatomen, für jeweils geradkettiges oder verzweigtes Halogenalkenyl oder Halogenalkinyl mit jeweils 3 bis 8 Kohlenstoffatomen und 1 bis 3 Halogenatomen, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen, für jeweils gegebenenfalls geradkettiges oder verzweigtes Methoximinoalkyl oder Ethoximinoalkyl mit jeweils 1 bis 5 Kohlenstoffatomen im Alkylteil, oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl stehen, wobei als Substituenten jeweils infrage kommen: Fluor, Chlor, Brom, Methyl, Ethyl, n-oder i-Propyl, n-, i-, s-oder t-Butyl, Cyano, Methandiyl, Ethandiyl, Butandiyl oder Butadiendiyl; $R^3$ und $R^4$ außerdem unabhängig voneinander für im Heterocyclylteil gegebenenfalls ein- bis dreifach, gleich oder verschieden substituiertes Heterocyclylmethyl, Heterocyclylpropyl, Heterocyclylethyl oder Heterocyclylbutyl stehen, wobei als Heterocyclen jeweils infrage kommen:

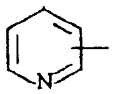 ; 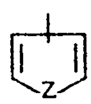 ; 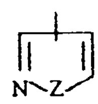 ; 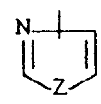 ;

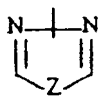 ; 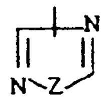 ; 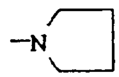 ; 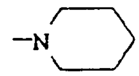 ;

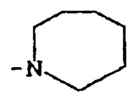 ; 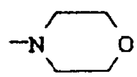 oder 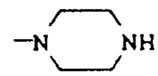

wobei Z jeweils für Sauerstoff oder Schwefel steht und wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio; $R^3$ und $R^4$ außerdem unabhängig voneinander für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein-bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl stehen, wobei als Phenylsubstituenten jeweils infrage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trilfuormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy; oder

$R^3$ und $R^4$ gemeinsam mit dem Stickstoffatom, an welches sie gebunden sind, für einen gegebenenfalls ein-bis dreifach, gleich oder verschieden substituierten Heterocyclus der Formel

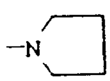 ; 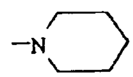 ; 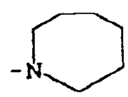 ; 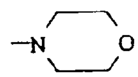 ;

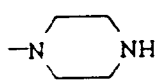 ; 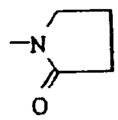 ; 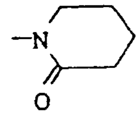 ; 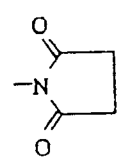 ;

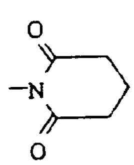 ; 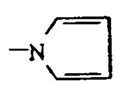 oder 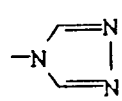

stehen,
wobei als Substituenten infrage kommen: Methyl, Ethyl, n- oder i-Propyl, Chlor oder Trifluormethyl,

X für Sauerstoff oder Schwefel steht und

Y für Sauerstoff oder Schwefel steht.

3. Verfahren zur Herstellung von substituierten Triazolonen der allgemeinen Formel (I),

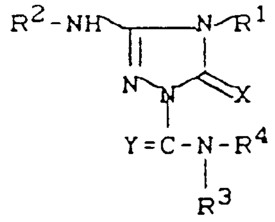

(I)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten infrage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl mit jeweils 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil, Aryl mit 6 bis 10 Kohlenstoffatomen steht, wobei als Substituenten im Arylteil jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^3$ und $R^4$ unabhängig voneinander jeweils für Wasserstoff, für jeweils geradkettiges oder verzweigtes Alkyl mit 1 bis 18 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, für Alkylaminoalkyl, Dialkylaminoalkyl oder Alkoximinoalkyl mit jeweils 1 bis 8 Kohlenstoffatomen in den einzelnen Alkylteilen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil stehen, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenene Halogenatomen oder jeweils zweifach verknüpftes Alkandiyl oder Alkendiyl mit jeweils bis zu 4 Kohlenstoffatomen; $R^3$ und $R^4$

außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil stehen, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^3$ und $R^4$ außerdem unabhängig voneinander für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aralkyloxy, Aryloxy, Aroyl oder Aryl mit jeweils 6 bis 10 Kohlenstoffatomen im Arylteil und gegebenenfalls 1 bis 6 Kohlenstoffatomen im Alkylteil stehen, wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen und gegebenenfalls 1 bis 9 gleichen oder verschiedenen Halogenatomen, Cycloalkyl mit 3 bis 6 Kohlenstoffatomen oder Phenoxy und wobei als Alkylsubstituenten gegebenenfalls infrage kommen: Halogen oder Cyano, oder

$R^3$ und $R^4$ gemeinsam mit dem Stickstoffatom, an welches sie gebunden sind, für einen gegebenenfalls einfach oder mehrfach, gleich oder verschieden substituierten fünf- bis zehngliedrigen Heterocyclus stehen, der gegebenenfalls 1 bis 2 weitere Heteroatome, insbesondere Stickstoff, Sauerstoff und/oder Schwefel, enthalten kann, wobei als Substituenten infrage kommen: Halogen sowie jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen sowie 1 bis 2 Oxo- oder Thionogruppen,

X für Sauerstoff oder Schwefel steht und

Y für Sauerstoff oder Schwefel steht, dadurch gekennzeichnet, daß man

a) 1-Chlor-(thio)carbonyltriazolone der Formel (II),

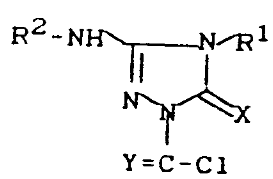

(II)

in welcher

$R^1$, $R^2$, X und Y die oben angegebene Bedeutung haben,

mit Aminen der Formel (III),

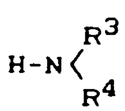

(III)

in welcher

$R^3$ und $R^4$ die oben angegebene Bedeutung haben,

gegebenenfalls in Gegenwart eines Verdünnungsmittels und gegebenenfalls in Gegenwart eines Säurebindemittels umsetzt, oder

b) für den Fall, daß in Formel (I) $R^3$ für Wasserstoff steht, daß man in 1-Stellung unsubstituierte Triazolone der Formel (IV),

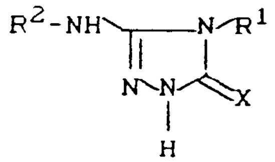

(IV)

in welcher
$R^1$, $R^2$ und X die oben angegebene Bedeutung haben,
mit Iso(thio)cyanaten der Formel (V),

$$R^4\text{-N=C=Y} \qquad (V)$$

in welcher
$R^4$ und Y die oben angegebene Bedeutung haben,
gegebenenfalls in Gegenwart eines Verdünnungsmittels und gegebenenfalls in Gegenwart eines Reaktionshilfsmittels umsetzt.

4. Herbizide Mittel, gekennzeichnet durch einen Gehalt an mindestens einem substituierten Triazolon der Formel (I) gemäß den Ansprüchen 1 oder 3.

5. Verfahren zur Bekämpfung von unerwünschtem Pflanzenwuchs, dadurch gekennzeichnet, daß man substituierte Triazolone der Formel (I) gemäß den Ansprüchen 1 oder 3 auf die Pflanzen und/oder ihren Lebensraum einwirken läßt.

6. Verwendung von substituierten Triazolonen der Formel (I) gemäß den Ansprüchen 1 oder 3 zur Bekämpfung von unerwünschten Pflanzen.

7. Verfahren zur Herstellung von herbiziden Mitteln, dadurch gekennzeichnet, daß man substituierte Triazolone der Formel (I) gemäß den Ansprüchen 1 oder 3 mit Streckmitteln und/oder oberflächenaktiven Substanzen vermischt.

8. 1-Chlor-(thio)carbonyltriazolone der Formel (II),

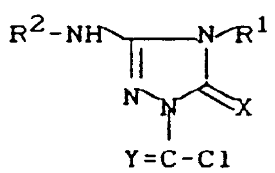

(II)

in welcher

$R^1$ für Wasserstoff, für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 2 bis 8 Kohlenstoffatomen und 1 bis 13 gleichen oder verschiedenen Halogenatomen, Alkoxyalkyl oder Alkoxy mit jeweils 1 bis 6 Kohlenstoffatomen in den einzelnen Alkylteilen, für Cycloalkylalkyl oder Cycloalkyl mit jeweils 3 bis 7 Kohlenstoffatomen im Cycloalkylteil und gegebenenfalls 1 bis 6 Kohlenstoffatomen im geradkettigen oder verzweigten Alkylteil oder für jeweils gegebenenfalls einfach oder mehrfach, gleich oder verschieden substituiertes Aralkyl oder Aryl mit jeweils 6 bis 10 Kohlenstoffatomen im Arylteil und gegebenenfalls 1 bis 6 Kohlenstoffatomen im geradkettigen oder verzweigten Alkylteil steht, wobei als Arylsubstituenten infrage kommen: Halogen, Cyano, Nitro, sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

$R^2$ für jeweils geradkettiges oder verzweigtes Alkyl mit 1 bis 8 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Alkoxyalkyl mit jeweils 1 bis 6 Kohlenstoffatomen in den einzelnen Alkylteilen, für Cycloalkyl mit 3 bis 7 Kohlenstoffatomen, für Cycloalkylalkyl mit 3 bis 7 Kohlenstoffatomen im Cycloalkylteil und 1 bis 6 Kohlenstoffatomen im Alkylteil oder für jeweils gegebenenfalls einfach oder mehrfach, gleich oder verschieden substituiertes Aralkyl mit 6 bis 10 Kohlenstoffatomen im Arylteil und 1 bis 6 Kohlenstoffatomen im Alkylteil, Aryl mit 6 bis 10 Kohlenstoffatomen steht, wobei als Substituenten im Arylteil jeweils infrage kommen: Halogen, Cyano, Nitro sowie jeweils geradkettiges oder verzweigtes Alkyl, Alkoxy, Alkylthio, Halogenalkyl, Halogenalkoxy oder Halogenalkylthio mit jeweils 1 bis 4 Kohlenstoffatomen und gegebenenfalls 1 bis 9 gleichen oder verschiedenen Halogenatomen,

X und Y unabhängig voneinander für Sauerstoff oder Schwefel stehen.

9. Verbindungen der Formel (IV)

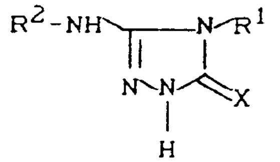

(IV)

in welcher

(α) X für Sauerstoff steht und $R^1$ und $R^2$ für Methyl stehen, oder in welcher

(β) X für Sauerstoff oder Schwefel steht und $R^1$ für Methyl und gleichzeitig $R^2$ für Ethyl steht oder $R^1$ für Ethyl und gleichzeitig $R^2$ für Methyl steht oder $R^1$ und $R^2$ für Ethyl stehen, ausgenommen die Verbindung 4-Ethyl-5-(ethylamino)-2,4-dihydro-1,2,4-triazol-3-thion.

## Claims

1. Substituted triazolones of the general formula (I)

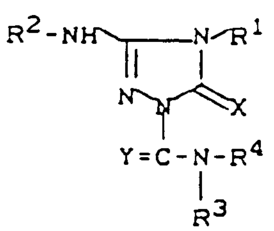

(I)

in which

$R^1$ represents hydrogen, or represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl or alkoxy, each of which has 1 to 6 carbon atoms in the individual alkyl moieties, or represents cycloalkylalkyl or cycloalkyl, each of which has 3 to 7 carbon atoms in the cycloalkyl moiety and, if appropriate, 1 to 6 carbon atoms in the straight-chain or branched alkyl moiety, or represents aralkyl or aryl, each of which has 6 to 10 carbon atoms in the aryl moiety and, if appropriate, 1 to 6 carbon atoms in the straight-chain or branched alkyl moiety and each of which is optionally monosubstituted or polysubstituted by identical or different substituents, suitable aryl substituents being: halogen, cyano, nitro, and in each case straight-chain or branched alkyl, alkoxy, alkylthio, halogenoalkyl, halogenoalkoxy or halogenoalkylthio, each of which has 1 to 4 carbon

atoms and, if appropriate, 1 to 9 identical or different halogen atoms,

$R^2$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogeno-alkenyl or halogenoalkinyl, each of which has 2 to 8 carbon atoms and 1 to 15, or 13, identical or different halogen atoms, alkoxyalkyl which has 1 to 6 carbon atoms in each of the individual alkyl moieties, or represents cyclo-alkyl having 3 to 7 carbon atoms, or represents cycloalkylalkyl having 3 to 7 carbon atoms in the cycloalkyl moiety and 1 to 6 carbon atoms in the alkyl moiety, or represents aralkyl which has 6 to 10 carbon atoms in the aryl moiety and 1 to 6 carbon atoms in the alkyl moiety and which is optionally monosubstituted or polysubstituted by identical or different substituents, aryl having 6 to 10 carbon atoms, suitable substituents in the aryl moiety in each case being: halogen, cyano, nitro and in each case straight-chain or branched alkyl, alkoxy, alkylthio, halogeno-alkyl, halogenoalkoxy or halogenoalkylthio, each of which has 1 to 4 carbon atoms and if appropriate 1 to 9 identical or different halogen atoms,

$R^3$ and $R^4$ independently of one another each represent hydrogen, or represent in each case straight-chain or branched alkyl having 1 to 18 carbon atoms, alkenyl having 2 to 8 carbon atoms, alkinyl having 2 to 8 carbon atoms, halogeno-alkyl having 1 to 8 carbon atoms and 1 to 17 identical or different halogen atoms, halogeno-alkenyl or halogenoalkinyl, each of which has 2 to 8 carbon atoms and 1 to 15, or 13, identical or different halogen atoms, cyano-alkyl having 1 to 8 carbon atoms, hydroxyalkyl having 1 to 8 carbon atoms and 1 to 6 hydroxyl groups, alkoxyalkyl, alkoxycarbonylalkyl or alkoxycarbonylalkenyl, each of which has up to 6 carbon atoms in the individual alkyl or alkenyl moieties, or represent alkylaminoalkyl, dialkylaminoalkyl or alkoximinoalkyl, each of which has 1 to 8 carbon atoms in the individual alkyl moieties, or represent cycloalkyl, cycloalkylalkyl, cycloalkenyl or cycloalkenylalkyl, each of which has 3 to 8 carbon atoms in the cycloalkyl or cycloalkenyl moiety and if appropriate 1 to 6 carbon atoms in the alkyl moiety and each of which is optionally mono-substituted or polysubstituted by identical or different substituents, suitable substituents in each case being: halogen, cyano and in each case straight-chain or branched alkyl or halogenoalkyl, each of which has 1 to 4 carbon atoms and, if appropriate, 1 to 9 identical or different halogen atoms, or in each case divalent alkanediyl or alkenediyl, each of which has up to 4 carbon atoms; $R^3$ and $R^4$ furthermore represent heterocyclylalkyl which has 1 to 6 carbon atoms in the straight-chain or branched alkyl moiety and 1 to 9 carbon atoms as well as 1 to 3 hetero atoms - in particular nitrogen, oxygen and/or sulphur - in the heterocyclyl moiety and which is optionally monosubstituted or polysubstituted in the heterocyclyl moiety by identical or different substituents, suitable substituents in each case being: halogen, cyano, nitro, and in each case straight-chain or branched alkyl, alkoxy, alkylthio, halogenoalkyl, halogenoalkoxy, halogenoalkylthio or alkoxycarbonyl, each of which has 1 to 5 carbon atoms and, if appropriate, 1 to 9 identical or different halogen atoms; $R^3$ and $R^4$ independently of one another furthermore represent in each case straight-chain or branched alkoxy having 1 to 8 carbon atoms, alkenyloxy having 2 to 8 carbon atoms or alkinyloxy having 2 to 8 carbon atoms and, finally, aralkyl, aralkyloxy, aryloxy, aroyl or aryl, each of which has 6 to 10 carbon atoms in the aryl moiety and, if appropriate, 1 to 6 carbon atoms in the alkyl moiety and each of which is optionally monosubstituted or polysubstituted

by identical or different substituents, suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl, each of which has 1 to 6 carbon atoms and if appropriate 1 to 9 identical or different halogen atoms, cycloalkyl having 3 to 6 carbon atoms or phenoxy, and, if appropriate, suitable alkyl substituents being: halogen or cyano, or

$R^3$ and $R^4$ together with the nitrogen atom to which they are bonded represent a five- to ten-membered heterocycle which, if appropriate, can contain 1 to 2 further heteroatoms, in particular nitrogen, oxygen and/or sulphur, and which is optionally monosubstituted or polysub-stituted by identical or different substituents, suitable substituents being: halogen and in each case straight-chain or branched alkyl or halogenoalkyl, each of which has 1 to 4 carbon atoms and if appropriate 1 to 9 identical or different halogen atoms and also 1 to 2 oxo or thiono groups,

X represents oxygen or sulphur and

Y represents oxygen or sulphur.

**2.** Substituted triazolones of the formula (I) according to Claim 1, in which

$R^1$ represents hydrogen, methyl, ethyl, n- or i-propyl, n-, i-, s- or t-butyl, n- or i-pentyl or n- or i-hexyl, or represents allyl, propargyl, methoxy, ethoxy or methoxymethyl, or represents straight-chain or branched halogenoalkyl having 1 to 4 carbon atoms and 1 to 9 identical or different halogen atoms, or represents cyclopentyl, cyclohexyl, cyclopropyl, cyclopropylmethyl, cyclohexylmethyl or cyclohexylethyl, or represents benzyl or phenyl, each of which is optionally monosubstituted to trisubstituted by identical or different substituents, suitable substituents being: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio,

$R^2$ represents methyl, ethyl, n- or i-propyl, n-, i-, s- or t-butyl, n- or i-pentyl, allyl or propargyl, or represents in each case straight-chain or branched halogenoalkyl having 1 to 4 carbon atoms, halogenoalkenyl having 3 to 6 carbon atoms or halogenoalkinyl having 3 to 6 carbon atoms and in each case 1 to 9 identical or different halogen atoms, or represents methoxymethyl or methoxyethyl, or represents cyclopropyl, cyclopropylmethyl, cyclopentyl, cyclohexyl, cyclohexylmethyl, cyclohexylethyl or cyclopentylmethyl, or represents benzyl, phenylethyl or phenyl, each of which is optionally monosubstituted or trisubstituted by identical or different substituents, suitable substituents in each case being: fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio,

$R^3$ and $R^4$ independently of one another each represent hydrogen, methyl, ethyl, n- or i-propyl, n-, i-, s- or t-butyl, n- or i-pentyl, n- or i-hexyl, n- or i-heptyl, n- or i-octyl, n- or i-nonyl, n- or i-decyl, n- or i-dodecyl, or represent allyl, n- or i-butenyl, n- or i-pentenyl, n- or i-hexenyl, propargyl, n- or i-butinyl, n- or i-pentinyl, n- or i-hexinyl, or represent straight-chain or branched halogenoalkyl having 1 to 6 carbon atoms and 1 to 9 identical or different halogen atoms, or represent in each case straight-chain or branched halogenoalkenyl or halogenoalkinyl, each of which has 3 to 8 carbon atoms and 1 to 3 halogen atoms, or represent in each case straight-chain or branched cyanoalkyl having 1 to 6 carbon atoms in the alkyl moiety, hydroxylalkyl having 1 to 6 carbon atoms and 1 to 3 hydroxyl groups, alkoxyalkyl, alkoxycarbonylalkyl or alkoxycarbonylalkenyl, alkylaminoalkyl or dialkylaminoalkyl, each of which has up to 4 carbon atoms in the individual alkyl or alkenyl moieties, or represent in each case optionally straight-chain or branched methoximinoalkyl or ethoximinoalkyl, each of which has 1 to 5 carbon atoms in the alkyl moiety, or represent cyclopropyl, cyclopropylmethyl, cyclopropylethyl, cyclopentyl, cyclohexyl, cycloheptyl, cyclooctyl, cyclohexylmethyl, cyclohexylethyl, cyclohexenyl or cyclohexenylmethyl, each of which is optionally monosubstituted to trisubstituted by identical or different substituents, suitable substituents in each case being: fluorine, chlorine, bromine, methyl, ethyl, n- or i-propyl, n-, i-, s- or t-butyl, cyano, methanediyl, ethanediyl, butanediyl or butadienediyl; $R^3$ and $R^4$ furthermore independently of one another represent heterocyclylmethyl, heterocyclylpropyl, heterocyclylethyl or heterocyclylbutyl, each of which is optionally monosubstituted to trisubstituted in the heterocyclyl moiety by identical or different substituents, suitable heterocycles in each case being:

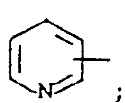 ; 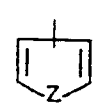 ; 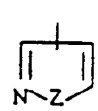 ; 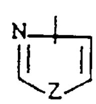 ;

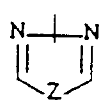 ; 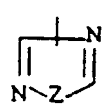 ; 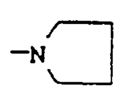 ; 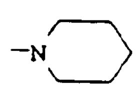 ;

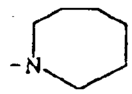 ; 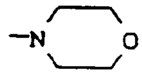 oder 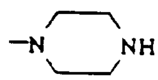

where Z in each case represents oxygen or sulphur, suitable substituents being fluorine, chlorine, bromine, cyano, nitro, methyl, ethyl, n- or i-propyl, n-, i-, s- or t-butyl, methoxy, ethoxy, methylthio, trifluoromethyl, trifluoromethoxy or trifluoromethylthio; $R^3$ and $R^4$ furthermore independently of one another represent in each case straight-chain or branched alkoxy having 1 to 6 carbon atoms, alkenyloxy having 3 to 6 carbon atoms or alkinyloxy having 3 to 6 carbon atoms, or represent optionally straight-chain or branched benzyl, phenylethyl, phenylpropyl, phenylbutyl, phenylpentyl, phenylhexyl, phenylheptyl, phenylcyanomethyl, phenylcyanoethyl, phenylcyanopropyl, benzyloxy, phenylethyloxy, phenoxy, benzoyl, phenyl or naphthyl, each of which is optionally monosubstituted to trisubstituted by identical or different substituents, suitable phenyl substituents in each case being: fluorine, chlorine, bromine, hydroxyl, cyano, nitro, methyl, ethyl, n- or i-propyl, n-, i-, s- or t-butyl, methoxy, ethoxy, methylthio, trifluoromethyl, trifluoromethoxy, trifluoromethylthio, trifluoromethylsulphinyl, trifluoromethylsulphonyl, methylsulphinyl, methylsulphonyl, acetyl, propionyl, methoxycarbonyl, ethoxycarbonyl, cyclohexyl or phenoxy; or

$R^3$ and $R^4$ together with the nitrogen atom to which they are bonded represent a heterocycle of the formula

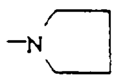 ; 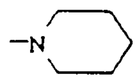 ; 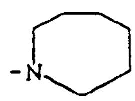 ; 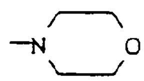 ;

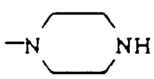 ; 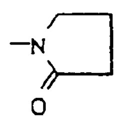 ; 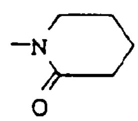 ; 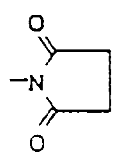 ;

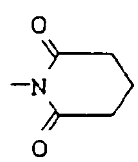 ; 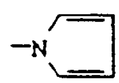 or 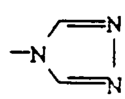

which is optionally monosubstituted to trisubstituted by identical or different substituents, suitable substituents in each case being: methyl, ethyl, n- or i-propyl, chlorine or trifluoromethyl,

X represents oxygen or sulphur and

Y represents oxgyen or sulphur.

3. Process for the preparation of substituted triazolones of the general formula (I)

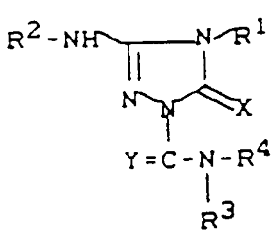

(I)

in which

$R^1$ represents hydrogen, or represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl or alkoxy, each of which has 1 to 6 carbon atoms in the individual alkyl moieties, or represents cycloalkylalkyl or cycloalkyl, each of which has 3 to 7 carbon atoms in the cycloalkyl moiety and, if appropriate, 1 to 6 carbon atoms in the straight-chain or branched alkyl moiety, or represents aralkyl or aryl, each of which has 6 to 10 carbon atoms in the aryl moiety and, if appropriate, 1 to 6 carbon atoms in the straight-chain or branched alkyl moiety and each of which is optionally monosubstituted or polysubstituted by identical or different substituents, suitable aryl substituents being: halogen, cyano, nitro, and in each case straight-chain or branched alkyl, alkoxy, alkylthio, halogenoalkyl, halogenoalkoxy or halogenoalkylthio, each of which has 1 to 4 carbon atoms and, if appropriate, 1 to 9 identical or different halogen atoms,

$R^2$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, each of which has 2 to 8 carbon atoms and 1 to 15, or 13, identical or different halogen atoms, alkoxyalkyl which has 1 to 6 carbon atoms in each of the individual alkyl moieties, or represents cycloalkyl having 3 to 7 carbon atoms, or represents cycloalkylalkyl having 3 to 7 carbon atoms in the cycloalkyl moiety and 1 to 6 carbon atoms in the alkyl moiety, or represents aralkyl which has 6 to 10 carbon atoms in the aryl moiety and 1 to 6 carbon atoms in the alkyl moiety and which is optionally monosubstituted or polysubstituted by identical or different substituents, aryl having 6 to 10 carbon atoms, suitable substituents in the aryl moiety in each case being: halogen, cyano, nitro and in each case straight-chain or branched alkyl, alkoxy, alkylthio, halogenoalkyl, halogenoalkoxy or halogenoalkylthio, each of which has 1 to 4 carbon atoms and if appropriate 1 to 9 identical or different halogen atoms,

$R^3$ and $R^4$ independently of one another each represent hydrogen, or represent in each case straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, each of which has 2 to 8 carbon atoms and 1 to 15, or 13, identical or different halogen atoms, cyanoalkyl having 1 to 8 carbon atoms, hydroxyalkyl having 1 to 8 carbon atoms and 1 to 6 hydroxyl groups, alkoxyalkyl, alkoxycarbonylalkyl or alkoxycarbonylalkenyl, each of which has up to 6 carbon atoms in the individual alkyl or alkenyl moieties, or represent alkylaminoalkyl, dialkylaminoalkyl or alkoximinoalkyl, each of which has 1 to 8 carbon atoms in the individual alkyl moieties, or represent cycloalkyl, cycloalkylalkyl, cycloalkenyl or cycloalkenylalkyl, each of which has 3 to 8 carbon atoms in the cycloalkyl or cycloalkenyl moiety and if appropriate 1 to 6 carbon atoms in the alkyl moiety and each of which is optionally mono-substituted or polysubstituted by identical or different substituents, suitable substituents in each case being: halogen, cyano and in each case straight-chain or branched alkyl or halogenoalkyl, each of which has 1 to 4 carbon atoms and, if appropriate, 1 to 9 identical or different halogen atoms, or in each case divalent alkanediyl or alkenediyl, each of which has up to 4 carbon atoms; $R^3$ and $R^4$ furthermore represent heterocyclylalkyl which has 1 to 6 carbon atoms in the straight-chain or branched alkyl moiety and 1 to 9 carbon atoms as well as 1 to 3 hetero atoms - in particular nitrogen, oxygen and/or sulphur - in the heterocyclyl moiety and which is optionally monosubstituted or polysubstituted in the heterocyclyl moiety by identical or different substituents, suitable substituents in each case being: halogen, cyano, nitro, and in each case straight-chain or branched alkyl, alkoxy, alkylthio, halogenoalkyl, halogenoalkoxy, halogenoalkylthio or alkoxycarbonyl, each of which has 1 to 5 carbon atoms and, if appropriate, 1 to 9 identical or different halogen atoms; $R^3$ and $R^4$ independently of one another furthermore represent in each case straight-chain or branched alkoxy having 1 to 8 carbon atoms, alkenyloxy having 2 to 8 carbon atoms or alkinyloxy having 2 to 8 carbon atoms and, finally, aralkyl, aralkyloxy, aryloxy, aroyl or aryl, each of which has 6 to 10 carbon atoms in the aryl

moiety and, if appropriate, 1 to 6 carbon atoms in the alkyl moiety and each of which is optionally monosubstituted or polysubstituted by identical or different substituents, suitable aryl substituents in each case being: halogen, cyano, nitro, hydroxyl, in each case straight-chain or branched alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl, each of which has 1 to 6 carbon atoms and if appropriate 1 to 9 identical or different halogen atoms, cycloalkyl having 3 to 6 carbon atoms or phenoxy, and, if appropriate, suitable alkyl substituents being: halogen or cyano, or

$R^3$ and $R^4$ together with the nitrogen atom to which they are bonded represent a five- to ten-membered heterocycle which, if appropriate, can contain 1 to 2 further heteroatoms, in particular nitrogen, oxygen and/or sulphur, and which is optionally monosubstituted or polysub-stituted by identical or different substituents, suitable substituents being: halogen and in each case straight-chain or branched alkyl or halogenoalkyl, each of which has 1 to 4 carbon atoms and if appropriate 1 to 9 identical or different halogen atoms and also 1 to 2 oxo or thiono groups,

X represents oxygen or sulphur and

Y represents oxygen or sulphur,

characterised in that

a) 1-chloro-(thio)carbonyltriazolones of the formula (II)

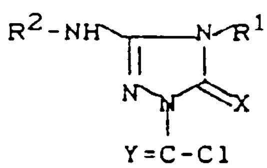

(II)

in which

$R^1$, $R^2$, X and Y are as defined above,

are reacted with amines of the formula (III)

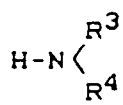

(III)

(III)

in which

$R^3$ and $R^4$ are as defined above,

if appropriate in the presence of a diluent and if appropriate in the presence of an acid-binding agent, or

b) in the event that $R^3$ in formula (I) represents hydrogen, in that triazolones which are unsubstituted in the 1-position, of the formula (IV)

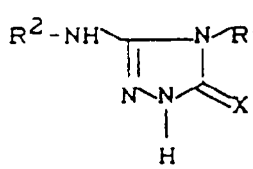

(IV)

in which

$R^1$, $R^2$ and X are as defined above,

are reacted with iso(thio)cyanates of the formula (V)

$$R^4\text{-N=C-Y} \qquad (V)$$

in which

$R^4$ and Y are as defined above,

if appropriate in the presence of a diluent and if appropriate in the presence of a reaction auxiliary.

4. Herbicidal agents, characterised in that they contain at least one substituted triazolone of the formula (I) according to Claims 1 or 3.

5. Method for combating undesired plant growth, characterised in that substituted triazolones of the formula (I) according to Claims 1 or 3 are allowed to act on the plants and/or their environment.

6. Use of substituted triazolones of the formula (I) according to Claims 1 or 3 for combating undesired plants.

7. Process for the preparation of herbicidal agents, characterised in that substituted triazolones of the formula (I) according to Claims 1 to 3 are mixed with extenders and/or surface-active substances.

8. 1-Chloro-(thio)carbonyltriazolones of the formula (II)

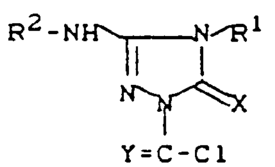

(II)

in which

$R^1$ represents hydrogen, or represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 2 to 8 carbon atoms and 1 to 13 identical or different halogen atoms, alkoxyalkyl or alkoxy, each of which has 1 to 6 carbon atoms in the individual alkyl moieties, or represents cycloalkylalkyl or cycloalkyl, each of which has 3 to 7 carbon atoms in the cycloalkyl moiety and, if appropriate, 1 to 6 carbon atoms in the straight-chain or branched alkyl moiety, or represents aralkyl or aryl, each of which has 6 to 10 carbon atoms in the aryl moiety and, if appropriate, 1 to 6 carbon atoms in the straight-chain or branched alkyl moiety and each of which is optionally monosubstituted or polysubstituted by identical or different substituents, suitable aryl substituents being: halogen, cyano, nitro, and in each case straight-chain or branched alkyl, alkoxy, alkylthio, halogenoalkyl, halogenoalkoxy or halogenoalkylthio, each of which has 1 to 4 carbon atoms and, if appropriate, 1 to 9 identical or different halogen atoms,

$R^2$ represents in each case straight-chain or branched alkyl having 1 to 8 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, each of which has 2 to 8 carbon atoms and 1 to 15, or 13, identical or different halogen atoms, alkoxyalkyl which has 1 to 6 carbon atoms in each of the individual alkyl moieties, or represents cycloalkyl having 3 to 7 carbon atoms, or represents cycloalkylalkyl having 3 to 7 carbon atoms in the cycloalkyl moiety and 1 to 6 carbon atoms in the alkyl moiety, or represents aralkyl which has 6 to 10 carbon atoms in the aryl moiety and 1 to 6 carbon atoms in the alkyl moiety and which is optionally monosubstituted or polysubstituted by identical or different substituents, aryl having 6 to 10 carbon atoms, suitable substituents in the aryl moiety in each case being: halogen, cyano, nitro and in each case straight-chain or branched alkyl, alkoxy, alkylthio, halogenoalkyl, halogenoalkoxy or halogenoalkylthio, each of which has 1 to 4 carbon atoms and if appropriate 1 to 9 identical or different halogen atoms, and

X and Y independently of one another represent oxgyen or sulphur.

9. Compounds of the formula (IV)

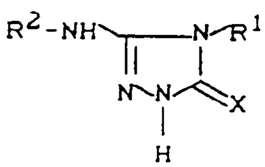

(IV)

in which

(α) X represents oxgyen and $R^1$ and $R^2$ represent methyl, or in which

(β) X represents oxygen or sulphur and $R^1$ represents methyl and, simultaneously, $R^2$ represents ethyl, or $R^1$ represents ethyl and, simultaneously, $R^2$ represents methyl, or $R^1$ and $R^2$ represent ethyl, with the exception of the compound 4-ethyl-5-(ethylamino)-2,4-dihydro-1,2,4-triazole-3-thione.

## Revendications

1. Triazolones substituées de formule générale (I)

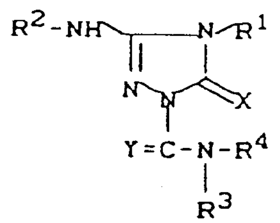

(I)

dans laquelle

$R^1$ représente un atome d'hydrogène, un groupe, chaque fois linéaire ou ramifié, alkyle ayant 1 à 8 atomes de carbone, alcényle ayant 2 à 8 atomes de carbone, alcynyle ayant 2 à 8 atomes de carbone, halogéno-alkyle ayant 1 à 8 atomes de carbone et 1 à 17 atomes d'halogène identiques ou différents, halogéno-alcényle ayant 2 à 8 atomes de carbone et 1 à 15 atomes d'halogène identiques ou différents, halogéno-alcynyle ayant 2 à 8 atomes de carbone et 1 à 13 atomes d'halogène identiques ou différents, alcoxyalkyle ou alcoxy ayant chacun 1 à 6 atomes de carbone dans les diverses parties alkyle, cycloalkylalkyle ou cycloalkyle ayant chaque fois 3 à 7 atomes de carbone dans la partie cycloalkyle et éventuellement 1 à 6 atomes de carbone dans la partie alkyle linéaire ou ramifiée, ou un groupe à chaque fois éventuellement substitué une ou plusieurs fois, de façon identique ou différente, aralkyle ou aryle ayant chacun 6 à 10 atomes de carbone dans la partie aryle et éventuellement 1 à 6 atomes de carbone dans la partie alkyle linéaire ou ramifiée, et l'on peut citer comme substituant du noyau aryle : un atome d'halogène, un reste cyano, nitro, ainsi qu'un reste, à chaque fois linéaire ou ramifié, alkyle, alcoxy, alkylthio, halogéno-alkyle, halogéno-alcoxy ou halogéno-alkylthio ayant chacun 1 à 4atomes de carbone et éventuellement 1 à 9atomes d'halogène identiques ou différents,

$R^2$ représente un groupe, à chaque fois linéaire ou ramifié, alkyle ayant 1 à 8 atomes de carbone, alcényle ayant 2 à 8 atomes de carbone, alcynyle ayant 2 à 8 atomes de carbone, halogéno-alkyle ayant 1 à 8 atomes de carbone et 1 à 17 atomes d'halogène identiques ou différents, halogéno-alcényle ou halogéno-alcynyle ayant chacun 2 à 8 atomes de carbone et 1 à 15 ou 13 atomes d'halogène identiques ou différents, alcoxyalkyle ayant chacun 1 à 6 atomes de carbone dans les diverses parties alkyles, un groupe cycloalkyle ayant 3 à 7 atomes de carbone, cycloalkylalkyle ayant 3 à 7 atomes de carbone dans la partie cycloalkyle et 1 à 6 atomes de carbone dans la partie alkyle ou un groupe aralkyle, chaque fois éventuellement substitué une ou plusieurs fois de façon identique ou différente et qui comporte 6 à 10 atomes de carbone dans la partie aryle et 1 à 6 atomes de carbone dans la partie alkyle, un groupe aryle ayant 6 à 10 atomes de carbone ; comme substituants dans la partie aryle, on peut citer chaque fois : un atome d'halogène, un reste cyano, nitro ainsi qu'un reste, chaque fois linéaire ou ramifié, alkyle, alcoxy, alkylthio, halogéno-alkyle, halogéno-alcoxy ou halogéno-alkylthio ayant chacun 1 à 4 atomes de carbone et éventuellement 1 à 9 atomes d'halogène identiques ou différents,

$R^3$ et $R^4$ représentent chacun, indépendamment l'un de l'autre, un atome d'hydrogène, un groupe à chaque fois linéaire ou ramifié, alkyle ayant 1 à 18 atomes de carbone, alcényle ayant 2 à 8 atomes de carbone, alcynyle ayant 2 à 8 atomes de carbone, halogéno-alkyle ayant 1 à 8 atome de carbone et 1 à 17 atomes d'halogène, identiques ou différents, halogéno-alcényle ou halogéno-alcynyle ayant chacun 2 à 8 atomes de carbone et 1 à 15 ou 13

atomes d'halogène identiques ou différents, cyanalkyle ayant 1 à 8 atomes de carbone, hydroxy-alkyle ayant 1 à 8 atomes de carbone et comportant 1 à 6 groupes hydroxy, alcoxyalkyle, alcoxycarbonylalkyle ou alcoxycarbonyl-alcényle ayant chacun jusqu'à 6 atomes de carbone dans les diverses parties alkyle ou alcényle, un groupe alkylaminoalkyle, dialkylaminoalkyle ou alcoxy-iminoalkyle ayant chaque fois 1 à 8 atomes de carbone dans les diverses parties alkyle ou un groupe chaque fois éventuellement substitué une ou plusieurs fois de manière identique ou différente, cycloalkyle, cycloalkyl-alkyle, cycloalcényle ou cycloalcénylalkyle ayant chacun 3 à 8 atomes de carbone dans la partie cycloalkyle ou cycloalcényle et éventuellement 1 à 6 atomes de carbone dans la partie alkyle, et l'on peut alors citer à chaque fois comme substituants : un atome d'halogène, un reste cyano, un reste, à chaque fois linéaire ou ramifié, alkyle ou halogéno-alkyle ayant chacun 1 à 4 atomes de carbone et éventuellement 1 à 9 atomes d'halogène identiques ou différents, ou bien un reste alcane-diyle ou alcène-diyle, chacun relié deux fois (divalent) portant chacun jusqu'à 4 atomes de carbone ; $R^3$ et $R^4$ peuvent en outre représenter chacun, indépendamment l'un de l'autre, un groupe hétéro-cyclyle alkyle (éventuellement substitué une ou plusieurs fois de façon identique ou différente dans la partie hétérocyclyle) comportant 1 à 4 atomes de carbone dans la partie alkyle linéaire ou ramifiée et comportant 1 à 9 atomes de carbone ainsi que 1 à 3 hétéroatomes, en particulier, l'azote, l'oxygène et/ou le soufre - dans la partie hétérocyclyle et l'on peut situer alors comme substituants : un atome d'halogène, un reste cyano, nitro ainsi qu'un reste, chaque fois linéaire ou ramifié, alkyle, alcoxy, alkylthio, halogéno-alkyle, halogéno-alcoxy, halogéno-alkylthio ou alcoxycarbonyle comportant chacun

1 à 5 atomes de carbone et éventuellement 1 à 9 atomes d'halogène identiques ou différents; $R^3$ et $R^4$ peuvent en outre, indépendamment l'un de l'autre, représenter un groupe, chaque fois linéaire ou ramifié, alcoxy ayant 1 à 8 atomes de carbone, alcényloxy ayant 2 à 8 atomes de carbone ou alcényloxy ayant 2 à 8 atomes de carbone et enfin un groupe, chaque fois éventuellement substitué une ou plusieurs fois de façon identique ou différente, aralkyle, aralkyloxy, aryloxy, aroyle ou aryle ayant chacun 6 à 10 atomes de carbone dans la partie aryle et éventuellement 1 à 6 atomes de carbone dans la partie alkyle, et l'on peut citer chaque fois comme substituant fixé sur la partie aryle: un atome d'halogène, un reste cyano, nitro, hydroxy, un reste, linéaire ou ramifié, alkyle, alcoxy, alkylthio, halogéno-alkyle, halogéno-alcoxy, halogéno-alkylthio, alkylsulfinyle, alkylsulfonyle, halogéno-alkylsulfinyle, halogéno-alkylsulfonyle, alcanoyle ou alcoxycarbonyle ayant chacun 1 à 6 atomes de carbone et comportant éventuellement 1 à 9 atomes d'halogène identiques ou différents, un reste cycloalkyle ayant 3 à 6 atomes de carbone ou phénoxy et, comme substituant du reste alkyle on peut éventuellement citer un atome d'halogène ou un reste cyano, ou bien

$R^3$ et $R^4$ forment ensemble, avec l'atome d'azote auquel ils sont reliés, un hétérocycle pentagonal à décagonal, éventuellement substitué une ou plusieurs fois de façon identique ou différente et qui peut contenir éventuellement 1 à 2 autres hétéroatomes, en particulier de l'azote, l'oxygène et/ou du soufre, et l'on peut citer comme substituants : un atome d'halogène ainsi qu'un reste, chaque fois linéaire ou ramifié, alkyle ou halogéno-alkyle ayant chacun 1 à 4 atomes de carbone et comportant éventuellement 1 à 9 atomes d'halogène identiques ou différents, ainsi que 1 ou 2 groupes oxo ou thiono,

X représente un atome d'oxygène ou de soufre, et

Y représente un atome d'oxygène ou de soufre.

2. Triazolones substituées de formule (I) selon la revendication 1, dans lesquelles

$R^1$ représente un atome d'hydrogène, un groupe méthyle, éthyle, n- ou i-propyle, n-, i-, s-, t-butyle, n-, i-pentyle, n-, i-hexyle, un groupe allyle, propargyle, méthoxy, éthoxy, méthoxyméthyle, un groupe halogéno-alkyle linéaire ou ramifié ayant 1 à 4 atomes de carbone et comportant 1 à 9 atomes d'halogène identiques ou différents, en particulier le fluor, le chlore ou le brome, un groupe cyclopentyle, cyclohexyle, cyclopropyle, cyclopropylméthyle, cyclohexylméthyle, cyclohexyléthyle ou un groupe benzyle ou phényle éventuellement substitué 1 à 3 fois, de façon identique ou différente, et on peut citer comme substituants : un atome de fluor, de chlore, de brome, un reste cyano, nitro, méthyle, éthyle, n-, i-propyle, n-, i-, s- ou t-butyle, méthoxy, éthoxy, méthylthio, trifluorométhyle, trifluorométhoxy ou trifluorométhylthio;

$R^2$ représente un groupe méthyle, éthyle, n- ou i-propyle, n-, i-, s- ou t-butyle, n- ou i-pentyle, allyle, propargyle, un groupe chaque fois linéaire ou ramifié, halogénoalkyle ayant 1 à 4 atomes de carbone, halogéno-alcényle ayant 3 à 6 atomes de carbone ou halogénoalcynyle ayant 3 à 6 atomes de carbone et comportant à chaque fois 1 à 9 atomes d'halogène identiques ou différents, un groupe méthoxyméthyle, méthoxyéthyle; un groupe cyclopropyle, cyclopropylméthyle, cyclopentyle, cyclohexyle, cyclohexylméthyle, cyclohexyléthyle, cyclopentylméthyle, un groupe, chaque fois éventuellement substitué 1 à 3 fois de façon identique ou différente, benzyle, phényléthyle ou phényle, et l'on peut alors citer comme substituants: un atome de fluor, de chlore, de brome, un reste cyano, nitro, méthyle, éthyle, n- ou i-propyle, n-, i-, s- ou t-butyle, méthoxy, éthoxy, méthylthio, trifluorométhyle, trifluorométhoxy ou trifluorométhylthio,

$R^3$ et $R^4$ représentent chacun, indépendamment l'un de l'autre, un atome d'hydrogène, un groupe méthyle, éthyle, n- ou i-propyle, n-, i-, s- ou t- butyle, n- ou i-pentyle, n- ou i-hexyle, n- ou i-heptyle, n- ou i-octyle, n- ou i-nonyle, n- ou i-décyle, n- ou i-dodécyle, un groupe allyle, n- ou i- butényle, n- ou i-pentényle, n- ou i-hexynyle, propargyle, n- ou i-butynyle, n- ou i-pentynyle, n- ou i-hexynyle, un groupe halogéno-alkyle linéaire ou ramifié comportant 1 à 6 atomes de carbone et 1 à 9 atomes d'halogène identiques ou différents, en particulier le fluor, le chlore ou le brome, un groupe halogéno-alcényle ou halogéno-alcynyle chaque fois linéaire ou ramifié et comportant à chaque fois 3 à 8 atomes de carbone et 1 à 3 atomes d'halogène, notamment le fluor ou le chlore, un groupe linéaire ou ramifié, cyanalkyle ayant 1 à 6 atomes de carbone dans la partie alkyle, hydroxyalkyle ayant 1 à 6 atomes de carbone et 1 à 3 groupes hydroxy, alcoxyalkyle, alcoxycarbonylalkyle ou alcoxycarbonylalcényle, alkylaminoalkyle ou dialkylaminoalkyle ayant chaque fois jusqu'à 4 atomes de carbone dans les diverses parties alkyle ou alcényle, un groupe, chaque fois linéaire ou ramifié, méthoxy-imino-alkyle ou éthoxy-imino-alkyle ayant chaque fois 1 à 5 atomes de carbone dans la partie alkyle, ou un groupe éventuellement substitué, à chaque fois 1 à 3 fois, de façon identique ou différente, cyclopropyle, cyclopropylméthyle, cyclopropyléthyle, cyclopentyle, cyclohexyle, cycloheptyle, cyclooctyle, cyclohexyméthyle, cyclohexyléthyle, cyclohexényle ou cyclohexénylméthyle, et l'on peut citer à chaque fois comme substituants : un atome de fluor, de chlore ou de brome, un reste méthyle, éthyle, n- ou i-propyle, n-, i-, s- ou t-butyle, cyano, méthanediyle, éthanediyle, butanediyle ou butadiènediyle ; $R^3$ et $R^4$ peuvent en outre représenter chacun, indépendamment l'un de l'autre, un groupe (éventuellement substitué 1 à 3 fois, de façon identique ou différente, dans la partie hétérocyclyle), hétérocyclylméthyle, hétérocyclylpropyle, hétérocyclyléthyle

ou hétérocyclylbutyle, et on peut citer à chaque fois comme hétérocycle:

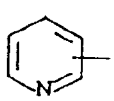 ; 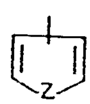 ; 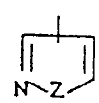 ; 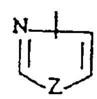 ;

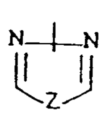 ; 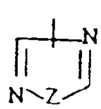 ; 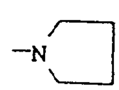 ; 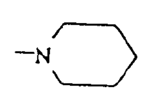 ;

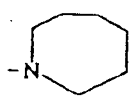 ; 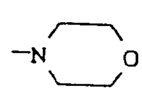 ou 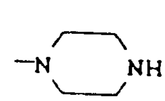

formules dans lesquelles Z représente à chaque fois un atome d'oxygène ou de soufre et l'on peut alors citer comme substituants : un atome de fluor, de chlore, de brome, un reste cyano, nitro, méthyle, éthyle, n-, i-propyle, n-, i-, s- ou t-butyle, méthoxy, éthoxy, méthylthio, trifluorométhyle, trifluorométhoxy ou trifluorométhylthio; $R^3$ et $R^4$ peuvent en

outre représenter chacun, indépendamment l'un de l'autre, un groupe, chaque fois linéaire ou ramifié, alcoxy ayant 1 à 6 atomes de carbone, alcényloxy ayant 3 à 6 atomes de carbone ou alcynyloxy ayant 3 à 6 atomes de carbone ou un groupe éventuellement substitué à chaque fois 1 à 3 fois de manière identique ou différente, éventuellement linéaire ou ramifié, benzyle, phényléthyle, phénylpropyle, phénylbutyle, phénylpentyle, phénylhexyle, phénylheptyle, phénylcyanométhyle, phénylcyano-éthyle, phénylcyanopropyle, benzyloxy, phényléthyloxy, phénoxy, benzoyle, phényle ou naphtyle, et l'on peut citer comme substituant des noyaux phényle, à chaque fois : un atome de fluor, de chlore, de brome, un reste hydroxy, cyano, nitro, méthyle, éthyle, n- ou i-propyle, n-, i-, s- ou t-butyle, méthoxy, éthoxy, méthylthio, trifluorométhyle, trifluorométhoxy, trifluorométhylthio, trifluorométhylsulfinyle, trifluorométhylsulfonyle, méthylsulfinyle, méthylsulfonyle, acétyle, propionyle, méthoxycarbonyle, éthoxycarbonyle, cyclohexyle ou phénoxy ; ou bien

$R^3$ et $R^4$ représentent ensemble, avec l'atome d'azote auquel ils sont reliés, un hétérocycle éventuellement substitué 1 à 3 fois, de façon identique ou différente, de formules

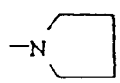 ; 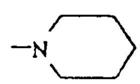 ; 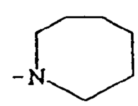 ; 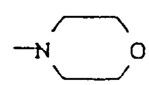 ;

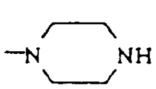 ; 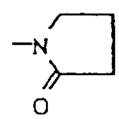 ; 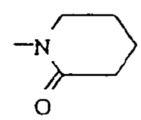 ; 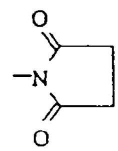 ;

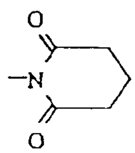 ; 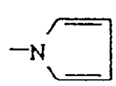 ou 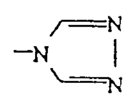 ,

et on peut citer comme substituants : un reste méthyle, éthyle, n- ou i-propyle, chloro ou trifluorométhyle,

X représente un atome d'oxygène ou de soufre, et

Y représente un atome d'oxygène ou de soufre.

3. Procédé pour préparer des triazolones substituées de formule générale (I):

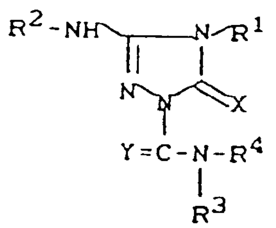

( I )

dans laquelle

$R^1$ représente un atome d'hydrogène, un groupe, chaque fois linéaire ou ramifié, alkyle ayant 1 à 8 atomes de carbone, alcényle ayant 2 à 8 atomes de carbone, alcynyle ayant 2 à 8 atomes de carbone, halogéno-alkyle ayant 1 à 8 atomes de carbone et 1 à 17 atomes d'halogène identiques ou différents, halogéno-alcényle ayant 2 à 8 atomes de carbone et 1 à 15 atomes d'halogène identiques ou différents, halogéno-alcynyle ayant 2 à 8 atomes de carbone et 1 à 13 atomes d'halogène identiques ou différents, alcoxyalkyle ou alcoxy ayant chacun 1 à 6 atomes de carbone dans les diverses parties alkyle, cyclo-alkylalkyle ou cycloalkyle ayant chaque fois 3 à 7 atomes de carbone dans la partie cycloalkyle et éventuellement 1 à 6 atomes de carbone dans la partie alkyle linéaire ou ramifiée, ou un

groupe à chaque fois éventuellement substitué une ou plusieurs fois, de façon identique ou différente, aralkyle ou aryle ayant chacun 6 à 10 atomes de carbone dans la partie aryle et éventuellement 1 à 6 atomes de carbone dans la partie alkyle linéaire ou ramifiée, et l'on peut citer comme substituant du noyau aryle : un atome d'halogène, un reste cyano, nitro, ainsi qu'un reste, à chaque fois linéaire ou ramifié, alkyle, alcoxy, alkylthio, halogéno-alkyle, halogéno-alcoxy ou halogéno-alkylthio ayant chacun 1 à 4atomes de carbone et éventuellement 1 à 9 atomes d'halogène identiques ou différents,

$R^2$ représente un groupe, à chaque fois linéaire ou ramifié, alkyle ayant 1 à 8 atomes de carbone, alcényle ayant 2 à 8 atomes de carbone, alcynyle ayant 2 à 8 atomes de carbone, halogéno-alkyle ayant 1 à 8 atomes de carbone et 1 à 17 atomes d'halogène identiques ou différents, halogéno-alcényle ou halogéno-alcynyle ayant chacun 2 à 8atomes de carbone et 1 à 15 ou 13 atomes d'halogène identiques ou différents, alcoxyalkyle ayant chacun 1 à 6 atomes de carbone dans les diverses parties alkyles, un groupe cycloalkyle ayant 3 à 7 atomes de carbone, cycloalkylalkyle ayant 3 à 7 atomes de carbone dans la partie cycloalkyle et 1 à 6 atomes de carbone dans la partie alkyle ou un groupe aralkyle, chaque fois éventuellement substitué une ou plusieurs fois de façon identique ou différente et qui comporte 6 à 10 atomes de carbone dans la partie aryle et 1 à 6 atomes de carbone dans la partie alkyle, un groupe aryle ayant 6 à 10 atomes de carbone ; comme substituants dans la partie aryle, on peut citer chaque fois : un atome d'halogène, un reste cyano, nitro ainsi qu'un reste, chaque fois linéaire ou ramifié, alkyle, alcoxy, alkylthio, halogéno-alkyle, halogéno-alcoxy ou halogéno-alkylthio ayant chacun 1 à 4 atomes de carbone et éventuellement 1 à 9 atomes d'halogène identiques ou différents,

$R^3$ et $R^4$ représentent chacun, indépendamment l'un de l'autre, un atome d'hydrogène, un groupe à chaque fois linéaire ou ramifié, alkyle ayant 1 à 18 atomes de carbone, alcényle ayant 2 à 8 atomes de carbone, alcynyle ayant 2 à 8 atomes de carbone, halogéno-alkyle ayant 1 à 8 atome de carbone et 1 à 17 atomes d'halogène, identiques ou différents, halogéno-alcényle ou halogéno-alcynyle ayant chacun 2 à 8 atomes de carbone et 1 à 15 ou 13 atomes d'halogène identiques ou différents, cyanalkyle ayant 1 à 8 atomes de carbone, hydroxy-alkyle ayant 1 à 8 atomes de carbone et comportant 1 à 6 groupes hydroxy, alcoxyalkyle, alcoxycarbonylalkyle ou alcoxycarbonylalcényle ayant chacun jusqu'à 6 atomes de carbone dans les diverses parties alkyle ou alcényle, un groupe alkylaminoalkyle, dialkylaminoalkyle ou alcoxy-iminoalkyle ayant chaque fois 1 à 8 atomes de carbone dans les diverses parties alkyle ou un groupe, chaque fois éventuellement substitué une ou plusieurs fois, de manière identique ou différente, cycloalkyle, cycloalkylalkyle, cycloalcényle ou cycloalcénylalkyle ayant chacun 3 à 8 atomes de carbone dans la partie cycloalkyle ou cycloalcényle et éventuellement 1 à 6 atomes de carbone dans la partie alkyle, et l'on peut alors citer à chaque fois comme substituants : un atome d'halogène, un reste cyano, un reste, à chaque fois linéaire ou ramifié, alkyle ou halogéno-alkyle ayant chacun 1 à 4 atomes de carbone et éventuellement 1 à 9 atomes d'halogène identiques ou différents, ou bien un reste alcane-diyle ou alcène-diyle, chacun relié deux fois (divalent) portant chacun jusqu'à 4 atomes de carbone ; $R^3$ et $R^4$ peuvent en outre représenter chacun, indépendamment l'un de l'autre, un groupe hétérocyclyle alkyle (éventuellement substitué une ou plusieurs fois de façon identique ou différente dans la partie hétérocyclyle) comportant 1 à 4 atomes de carbone dans la partie alkyle linéaire ou ramifiée et comportant 1 à 9 atomes de carbone ainsi que 1 à 3 hétéroatomes, en particulier, l'azote, l'oxygène et/ou le soufre - dans la partie hétérocyclyle et l'on peut citer alors comme substituants : un atome d'halogène, un reste cyano, nitro ainsi qu'un reste, chaque fois linéaire ou ramifié, alkyle, alcoxy, alkylthio, halogénoalkyle, halogéno-alcoxy, halogéno-alkylthio ou alcoxycarbonyle comportant chacun 1 à 5 atomes de carbone et éventuellement 1 à 9 atomes d'halogènes identiques ou différents ; $R^3$ et $R^4$ peuvent en outre, indépendamment l'un de l'autre, représenter un groupe, chaque fois linéaire ou ramifié, alcoxy ayant 1 à 8 atomes de carbone, alcényloxy ayant 2 à 8 atomes de carbone ou alcényloxy ayant 2 à 8 atomes de carbone et enfin un groupe, chaque fois éventuellement substitué une ou plusieurs fois, de façon identique ou différente, aralkyle, aralkyloxy, aryloxy, aroyle ou aryle ayant chacun 6 à 10 atomes de carbone dans la partie aryle et éventuellement 1 à 6 atomes de carbone dans la partie alkyle, et l'on peut citer chaque fois comme substituant fixé sur la partie aryle: un atome d'halogène, un reste cyano, nitro, hydroxy, un reste, linéaire ou ramifié, alkyle, alcoxy, alkylthio, halogéno-alkyle, halogéno-alcoxy, halogéno-alkylthio, alkylsulfinyle, alkylsulfonyle, halogéno-alkylsulfinyle, halogéno-alkylsulfonyle, alcanoyle ou alcoxycarbonyle ayant chacun 1 à 6 atomes de carbone et comportant éventuellement 1 à 9 atomes d'halogène identiques ou différents, un reste cycloalkyle ayant 3 à 6 atomes de carbone ou phénoxy et, comme substituant du reste alkyle, on peut éventuellement citer un atome d'halogène ou un reste cyano, ou bien

$R^3$ et $R^4$ forment ensemble, avec l'atome d'azote auquel ils sont reliés, un hétérocycle pentagonal à décagonal, éventuellement substitué une ou plusieurs fois de façon identique ou différente et qui peut contenir éventuellement 1 à 2 autres hétéroatomes, en particulier de l'azote, l'oxygène et/ou du soufre, et l'on peut citer comme substituants : un atome d'halogène ainsi qu'un reste, chaque fois linéaire ou ramifié, alkyle ou halogéno-alkyle ayant chacun 1 à 4 atomes de carbone et comportant éventuellement 1 à 9 atomes d'halogène identiques ou différents, ainsi que 1 ou 2 groupes oxo ou thiono,

X représente un atome d'oxygène ou de soufre, et

Y représente un atome d'oxygène ou de soufre.

procédé caractérisé en ce que:

a) on fait réagir des 1-chloro-(thio)carbonyltriazolones de formule (II),

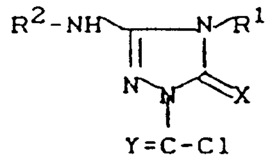

(II)

dans laquelle
$R^1$, $R^2$, X et Y ont le sens indiqué ci-dessus,
avec des amines de formule (III)

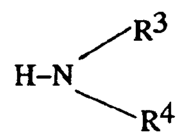

(III)

dans laquelle
$R^3$ et $R^4$ ont le sens indiqué ci-dessus,
éventuellement en présence d'un diluant et éventuellement en présence d'un agent de fixation des acides, ou

b) au cas où, dans la formule (I), $R^3$ représente un atome d'hydrogène, on fait réagir des triazolones non substituées en position 1 et répondant à la formule (IV),

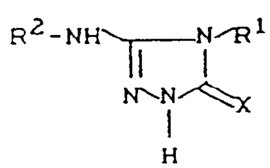

(IV)

dans laquelle
$R^1$, $R^2$ et X ont le sens indiqué ci-dessus,
avec des iso(thio)cyanates de formule (V),

$$R^4\text{-N=C=Y} \qquad (V)$$

dans laquelle
$R^4$ et Y ont le sens indiqué ci-dessus,
éventuellement en présence d'un diluant et éventuellement en présence d'un adjuvant de réaction.

**4.** Produit herbicide, caractérisé en ce qu'il contient au moins une triazolone substituée de formule (I) selon les revendications 1 ou 3.

**5.** Procédé pour combattre la croissance de plantes non souhaitée, caractérisé en ce qu'on fait agir sur les plantes et/ou sur leur biotope ou espace vital des triazolones substituées de formule (I) selon les revendications 1 ou 3.

**6.** Utilisation de triazolones substituées de formule (I) selon les revendications 1 ou 3 pour combattre des plantes non souhaitées.

**7.** Procédé pour fabriquer des produits herbicides, caractérisé en ce que l'on mélange des triazolones substituées, de formule (I) selon les revendications 1 ou 3, avec des agents d'allongement et/ou avec des substances tensioactives.

**8.** 1-chloro-(thio)carbonyltriazolones de formule (II)

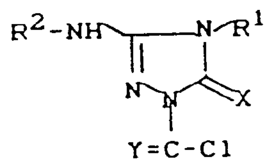

(II)

dans laquelle

$R^1$ représente un atome d'hydrogène, un groupe, chaque fois linéaire ou ramifié, alkyle ayant 1 à 8 atomes de carbone, alcényle ayant 2 à 8 atomes de carbone, alcynyle ayant 2 à 8 atomes de carbone, halogéno-alkyle ayant 1 à 8 atomes de carbone et 1 à 17 atomes d'halogène identiques ou différents, halogéno-alcényle ayant 2 à 8 atomes de carbone et 1 à 15 atomes d'halogène identiques ou différents, halogéno-alcynyle ayant 2 à 8 atomes de carbone et 1 à 13 atomes d'halogène identiques ou différents, alcoxyalkyle ou alcoxy ayant chacun 1 à 6 atomes de carbone dans les diverses parties alkyles, cycloalkylalkyles ou cycloalkyle ayant chaque fois 3 à 7 atomes de carbone dans la partie cycloalkyle et éventuellement 1 à 6 atomes de carbone dans la partie alkyle linéaire ou ramifiée, ou un groupe à chaque fois éventuellement substitué une ou plusieurs fois, de façon identique ou différente, aralkyle ou aryle ayant chacun 6 à 10 atomes de carbone dans la partie aryle et éventuellement 1 à 6 atomes de carbone dans la partie alkyle linéaire ou ramifiée, et l'on peut citer comme substituant du noyau aryle : un atome d'halogène, un reste cyano, nitro, ainsi qu'un reste, à chaque fois linéaire ou ramifié, alkyle, alcoxy, alkylthio, halogéno-alkyle, halogéno-alcoxy ou halogéno-alkylthio ayant chacun 1 à 4 atomes de carbone et éventuellement 1 à 9 atomes d'halogène identiques ou différents,

$R^2$ représente un groupe, à chaque fois linéaire ou ramifié, alkyle ayant 1 à 8 atomes de carbone, alcényle ayant 2 à 8 atomes de carbone, alcynyle ayant 2 à 8 atomes de carbone, halogéno-alkyle ayant 1 à 8 atomes de carbone et 1 à 17 atomes d'halogène identiques ou différents, halogéno-alcényle ou halogéno-alcynyle ayant chacun 2 à 8 atomes de carbone et 1 à 15 ou 13 atomes d'halogène identiques ou différents, alcoxyalkyle ayant chacun 1 à 6 atomes de carbone dans les diverses parties alkyles, un groupe cycloalkyle ayant 3 à 7 atomes de carbone, cycloalkylalkyle ayant 3 à 7 atomes de carbone dans la partie cycloalkyle et 1 à 6 atomes de carbone dans la partie alkyle ou un groupe aralkyle, chaque fois éventuellement substitué une ou plusieurs fois de façon identique ou différente et qui comporte 6 à 10 atomes de carbone dans la partie aryle et 1à 6 atomes de carbone dans la partie alkyle, un groupe aryle ayant 6 à 10 atomes de carbone ; comme substituants dans la partie aryle, on peut citer chaque fois : un atome d'halogène, un reste cyano, nitro ainsi qu'un reste, chaque fois linéaire ou ramifié, alkyle, alcoxy, alkylthio, halogéno-alkyle, halogéno-alcoxy ou halogéno-alkylthio ayant chacun 1 à 4 atomes de carbone et éventuellement 1 à 9 atomes d'halogène identiques ou différents,

X et Y représentent chacun, indépendamment l'un de l'autre, un atome d'hydrogène ou de soufre.

**9.** Composés de formule (IV)

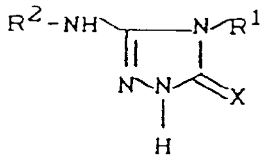

(IV)

dans laquelle

(α) X représente un atome d'oxygène et $R^1$ et $R^2$ représentent chacun groupe méthyle, ou bien

(β) X représente un atome d'oxygène ou de soufre, et $R^1$ représente un groupe méthyle et en même temps $R^2$ représente un groupe éthyle, ou bien $R^1$ représente un groupe éthyle et en même temps $R^2$ représente un groupe méthyle, ou bien $R^1$ et $R^2$ représentent chacun un groupe éthyle,

à l'exclusion du composé 4-éthyl-5-(éthylamino)-2,4-dihydro-1,2,4-triazole-3-thione.